# EUROPEAN PATENT APPLICATION

(11) **EP 4 814 023 A1**
(43) Date of publication of application: **30.09.2026**
(21) Application number: 24894221.1
(22) Date of filing: 22.11.2024
(51) Int. Cl.: C08F 20/06, C07B 61/00, C07C 1/20, C07C 11/06, C07C 45/29, C07C 49/08, C07C 51/25, C07C 57/05, C08F 8/00, C12P 7/06

(54) **METHOD FOR PRODUCING WATER-ABSORBING RESIN DERIVED FROM BIO-RAW MATERIALS, AND WATER-ABSORBING RESIN OBTAINED USING SAID METHOD**

(30) Priority: 22.11.2023 JP 2023198629; 28.12.2023 JP 2023223208
(71) Applicant: Nippon Shokubai Co., Ltd., Osaka-shi, Osaka 541-0043 (JP)
(72) Inventor: ISHIZAKI, Kunihiko, Himeji-shi, Hyogo 671-1282 (JP); KIKUCHI, Keisuke, Himeji-shi, Hyogo 671-1282 (JP); OKAMURA, Junji, Himeji-shi, Hyogo 671-1282 (JP); IGATA, Nao, Himeji-shi, Hyogo 671-1282 (JP); HAYASHI, Takumi, Himeji-shi, Hyogo 671-1282 (JP); KIRISHIKI, Masaru, Himeji-shi, Hyogo 671-1282 (JP)
(74) Representative: Hoffmann Eitle
(86) International application number: PCT/JP2024/041400
(87) International publication number: WO 2025/110231

(57) **Abstract**

Problem to be solved: it is an object to produce, at low cost, a water-absorbent resin having equivalent or higher performance and an equivalent or reduced amount of impurities as compared to a known water-absorbent resin derived from a fossil raw material, using a renewable biological raw material in production of a water-absorbent resin. Solution: a method for producing a water-absorbent resin derived from a biological raw material, the method including the following steps (i) to (7): (i) producing acetone from bioethanol; (ii) producing isopropanol from the acetone; (iii) producing propylene from the isopropanol (iii); (iv) producing acrylic acid from the propylene; (v) producing polyacrylic acid and/or a salt thereof by polymerizing an aqueous monomer solution containing the acrylic acid; (vi) drying the polyacrylic acid and/or a salt thereof; and (vii) performing surface-crosslinking on the polyacrylic acid and/or a salt thereof.

## Description

### Technical Field

The present invention relates to a method for producing a water-absorbent resin. More specifically, the present invention relates to a method for producing, with high productivity and at low cost, a water-absorbent resin having equivalent or higher performance and fewer impurities as compared to a water-absorbent resin derived from a fossil raw material using a renewable biological raw material.

### Background Art

The water-absorbent resin is also called a superabsorbent resin, a superabsorbent polymer (SAP), an absorbent gel material (AGM), a polymeric water-absorbing agent, a water-swellable polymer, or the like, and is widely used in hygienic materials such as disposable diapers, sanitary napkins, and so-called incontinence pads, as well as agricultural and horticultural water-retaining agents, air fresheners, deodorants, dehumidifying agents, cable water blocking materials, and the like. As water-absorbent resins, crosslinked products of partially neutralized polyacrylic acid, a starch-acrylic acid graft polymer, and the like are known. As typical polymerization methods for water-absorbent resins, aqueous solution polymerization, reverse phase suspension polymerization, and gas phase polymerization are known. For example, in the aqueous solution polymerization, the production is performed by carrying out a step of preparing an aqueous monomer solution with acrylic acid or the like, a polymerization step, a gel grinding step, a drying step, a sizing step (pulverization/classification), a fine powder recycling step, a surface-crosslinking step, an additive addition step, and the like (Non Patent Literature 1).

A water-absorbent resin is usually a synthetic polymer typified by a crosslinked product of polyacrylic acid salt. The monomer acrylic acid is commonly produced by oxidation of propylene produced by cracking naphtha from petroleum which is a fossil raw material. Water-absorbent resins are used mainly for hygienic materials such as disposable diapers, which are discarded in large amounts, and thus disposed off as used hygienic materials. Such polyacrylic acid salt water-absorbent resins derived from fossil raw materials have been recently considered as petrochemical products having an impact on the environment from the viewpoint of sustainability. To respond to this, a water-absorbent resin in which a polyacrylic acid salt is grafted to or mixed with a natural polymer (Patent Literature 17), a water-absorbent resin derived from a natural polymer, which is produced by crosslinking or carboxy-modifying a natural polymer (Patent Literature 18), and the like have been proposed instead of known water-absorbent resins of polyacrylic acid salts derived from a fossil raw materials (petroleum).

However, in the case of water-absorbent resins of natural polymers (for example, starch-grafted or starch-mixed polyacrylic acid crosslinked products, modified starch crosslinked products, carboxymethylcellulose crosslinked products, and polyamino acid crosslinked products), not only the heat resistance of the natural polymer is low, so that there is a problem of coloration (yellowing or browning of products because of the low heat resistance) in the production process for a water-absorbent resin, and processing under a high temperature condition is difficult, so that productivity is low, but also the use of the natural polymer leads to significant inferiority in water absorption performance as compared to known polyacrylic acid salt water-absorbent resins.

To address this, a water-absorbent resin having, as a monomer, acrylic acid derived from a biological raw material (bioacrylic acid) has been proposed in which the acrylic acid, which has been typically produced from a fossil raw material, is produced from a biological raw material (Patent Literatures 1 to 16, 19, and 20), instead of a method using, as a water-absorbent resin, a natural polymer that is poor in performance and heat resistance. In the case of a water-absorbent resin of acrylic acid derived from a biological raw material, a known production process for polyacrylic acid water-absorbent resins can be used as is, and thus there is an advantage that there are fewer problems in performance, productivity, and heat resistance (coloration) as compared to a water-absorbent resin in which a natural polymer is used.

Specifically, as a method for producing a water-absorbent resin via bioacrylic acid, a method using acrylic acid derived from glycerin (Patent Literatures 1 to 4), a method using acrylic acid produced by dehydrating lactic acid or 3-hydroxypropionic acid (Patent Literatures 5 to 8 and 20), a method using acrylic acid produced by dehydrating polyhydroxypropionic acid (Patent Literature 9), a method for producing acrylic acid from β-propiolactone (Patent Literature 10), a method using bionaphtha based on natural oil and/or fat (Patent Literatures 11 and 12), and the like are known. A method is also known in which for the water-absorbent resins, the content of a raw material derived from a biological raw material or identity as a water-absorbent resin in which bioacrylic acid is used is defined by the amount of carbon isotope ¹⁴C or ¹³C (Patent Literatures 7, 13, and 14).

However, in a method for producing bioacrylic acid without using propylene (Patent Literatures 1 to 10, 19, and 20), existing acrylic acid production equipment using fossil raw material propylene as a starting material cannot be used, and completely new production equipment is required, so that the acrylic acid is likely to be expensive from the viewpoint of the equipment cost. In production of bioacrylic acid, a biological raw material (lactic acid, 3-hydroxypropionic acid, glycerin, and bionaphtha based on natural oil and/or fat in Patent Literatures 1 to 14 and 20), which is more expensive than fossil raw material propylene, is used, but it is known that the amount of impurities is likely to increase as compared to that in acrylic acid derived from a fossil raw material, and for example, when biopropylene is used, thermal decomposition of bionaphtha is accompanied by a large amount of propane contained in propylene, and an increased amount of propane in biopropylene causes an increased amount of non-polymerizable propionic acid in acrylic acid produced by an oxidation reaction (Patent Literature 11), and biopropane is likely to contain sulfur, phosphorus, and nitrogen derived from a biological raw material (Patent Literature 12). Even without being via biopropylene, bioacrylic acid contains a relatively large amount of formic acid (Patent Literature 15), bioacrylic acid derived from glycerin contains a relatively large amount of hydroxyacetone, so that a water-absorbent resin is likely to be colored (Patent Literature 16), organic acids such as propionic acid, acetic acid and formic acid are formed as by-products, with propionic acid being particularly easily formed as a by-product via propionaldehyde, when acrylic acid is produced from glycerin, and there is a problem that organic acids which are fermentation by-products are incorporated when acrylic acid is produced from 3-hydroxypropionic acid. In particular, propionic acid and acrylic acid that are formed as by-products have almost the same boiling point of 141°C, and thus are difficult to separate. Removal of organic acids such as propionic acid from acrylic acid requires repeated purification, and even when purification is repeated at the sacrifice of yield and cost, removal of organic acid, in particular, propionic acid is not always sufficient.

As compared to relevant known acrylic acid derived from a fossil raw material, the increase or new generation of impurities in acrylic acid derived from bioacrylic acid causes not only purification cost and a decreased yield for bioacrylic acid, but also problems associated with the use of bioacrylic acid having the impurities for a water-absorbent resin (odor, coloration, performance deterioration, and the like of the water-absorbent resin).

Thus, at present, the production cost of any bioacrylic acid is high, and a polyacrylic acid (salt) water-absorbent resin with bioacrylic acid is also expensive. When the starting biological raw material is expensive or the production amount thereof is limited, there is a limit for replacement of water-absorbent resins derived from fossil raw materials, which are consumed in large amounts, by water-absorbent resins derived from biological raw materials. The polyacrylic acid (salt) water-absorbent resin derived from bioacrylic acid, when compared to a water-absorbent resin from acrylic acid derived from a fossil raw material, which is also a polyacrylic acid (salt) water-absorbent resin, has respective specific impurities of bioacrylic acid (for example, an increase of propionic acid in acrylic acid), and therefore tends to be inferior in odor (for example, acid odor of propionic acid), coloration and the like of the water-absorbent resin, which is ascribable to the impurities.

### Citation List

### Patent Literature

Patent Literature 1: WO 2006/092272
Patent Literature 2: WO 2006/136336
Patent Literature 3: WO 2008/023040
Patent Literature 4: WO 2010/066513
Patent Literature 5: WO 2006/092271
Patent Literature 6: WO 2008/023039
Patent Literature 7: WO 2007/109128
Patent Literature 8: WO 2013/155292
Patent Literature 9: WO 2013/185009
Patent Literature 10: WO 2018/085254
Patent Literature 11: WO 2014/079785
Patent Literature 12: JP 2018-083866 A
Patent Literature 13: WO 2011/136237
Patent Literature 14: WO 2011/136238
Patent Literature 15: WO 2011/040575
Patent Literature 16: WO 2009/130915
Patent Literature 17: WO 2007/098932
Patent Literature 18: WO 2002/096953
Patent Literature 19: WO 2010/090324
Patent Literature 20: WO 2010/090322

### Non-Patent Literature

Non-Patent Literature 1: Modern Superabsorbent Polymer Technology (1998); pp. 69 to 117

### Summary of Invention

### Technical Problem

An object is to obtain, at low cost, a water-absorbent resin having equivalent or higher performance and an equivalent or reduced amount of impurities as compared to a known water-absorbent resin derived from a fossil raw material, using a renewable biological raw material in production of a water-absorbent resin.

### Solution to Problem

In order to solve the above problems, the present inventors have focused on ethanol for the first time as a monomer starting material forming a backbone of a water-absorbent resin, and conducted studies to obtain a water-absorbent resin from ethanol which has been typically used mainly as a fuel, as a method for producing a water-absorbent resin derived from a biological raw material, instead of a method using, as a water-absorbent resin, a natural polymer that is poor in performance and heat resistance, and a known and typical method for producing bioacrylic acid (a starting material is glycerin, bionaphtha, lactic acid, 3-hydroxypropionic acid, bionaphtha based on natural oil and/or fat, or the like).

That is, the present inventors have conceived a method for reducing impurities (the content of propionic acid, and the like) in bioacrylic acid by suppressing production of propane and the like by synthesizing propylene by carrying out specific steps using, as a starting material, bioethanol that is produced in a large amount, instead of known and expensive biological raw materials (and biological raw materials whose production amount is limited).

In the present invention, it has been found that when bioacrylic acid produced from bioethanol by a specific step (bioethanol → bioacetone → bioisopropanol → biopropylene → bioacrylic acid) is used as a monomer, the bioacrylic acid derived from bioethanol and produced through specific steps does not cause problems (for example, coloration and odor) originating from water absorption performance and impurities, and is as preferred as or more preferred than known acrylic acid derived from a fossil raw material as a raw material acrylic acid for water-absorbent resins. In this way, the present invention has been completed by solving the above problems.

That is, the present invention provides a method for producing a water-absorbent resin derived from a biological raw material, the method including the following steps (i) to (vii) of:
(i) producing acetone from bioethanol;
(ii) producing isopropanol from the acetone;
(iii) producing propylene from the isopropanol;
(iv) producing acrylic acid from the propylene;
(v) producing polyacrylic acid and/or a salt thereof by polymerizing an aqueous monomer solution containing the acrylic acid;
(vi) drying the polyacrylic acid and/or a salt thereof; and
(vii) performing surface-crosslinking on the polyacrylic acid and/or a salt thereof.

The present invention provides a water-absorbent resin produced by the production method.

### Advantageous Effects of Invention

A water-absorbent resin having equivalent or higher performance and an equivalent or reduced amount of impurities as compared to a known water-absorbent resin derived from a fossil raw material can be produced at low cost using a renewable biological raw material in production of a water-absorbent resin. Biomass has already absorbed CO₂ in the air in the process of production thereof. Therefore, by producing a water-absorbent resin using bioethanol as a raw material, a carbon-neutral water-absorbent resin having high performance can be produced. Bioethanol is also a biological raw material that is produced in a large amount at low cost, and therefore, the water-absorbent resin of the present invention which is produced from bioethanol can be widely used as a water-absorbent resin derived from a biological raw material, instead of a water-absorbent resin derived from a fossil raw material that is consumed in a large amount.

### Description of Embodiments

### Description of Terms

### Biological Raw Material (Biomass)

In the present invention, the biological raw material (biomass) is an organic resource of biological origin, and may be a biological raw material of animal origin (for example, wool), but a renewable plant raw material is preferably used. Specifically, a biological raw material produced using, as a starting material, a plant component containing, for example, a natural polymer such as a saccharide, starch or cellulose is preferably used.

In the present invention, bioethanol, bioacetone, bioisopropanol, biopropylene and bioacrylic acid mean ethanol, acetone, isopropanol (also referred to as 2-propanol or isopropyl alcohol), propylene and acrylic acid with a biological raw material used as a raw material thereof or a further upstream raw material thereof, and are not different in chemical structure from known ethanol, acetone, isopropanol, propylene and acrylic acid except for the amount of carbon isotope ¹⁴C.

Hereinafter, ethanol, acetone, isopropanol, propylene and acrylic acid produced from biological raw materials may be referred to as bioethanol, bioacetone, bioisopropanol, biopropylene and bioacrylic acid, respectively, in order to emphasize that they are produced from biological raw materials.

In the present invention, the water-absorbent resin derived from a biological raw material means that a monomer derived from a biological raw material is contained as a monomer forming a backbone of the water-absorbent resin.

In the present invention, terms related to the performance of the water-absorbent resin are defined by the Worldwide Strategic Partners (WSP) standards of EDANA Recommended Test Methods, 2005 edition, unless otherwise specified. Examples of the performance defined by WSP include "pH" (WSP200.2), "residual monomer" (WSP210.2), "particle size distribution" (WSP220.2), "loss on drying" (WSP230.2), "free swelling capacity" or "FSC" (WSP240.2), "centrifugal retention capacity" or "CRC" (WSP241.2), "AUP (absorption under pressure)" or "AAP (absorption against pressure)" (WSP242.2), "permeability-dependent absorption under pressure" or "PDAUP" (WSP243.1), "flow rate" (WSP250.2), "bulk specific gravity" or "density" (WSP260.2), "water-soluble content" or "extractables" (WSP 270.2), absorbed particles (WSP280.2), and dust (WSP 290.2).

In the present invention, AAP may be measured under a pressure of 0.7 psi (4.8 kPa), and the AAP in this case may be referred to as AAP0.7.

In the present invention, the "moisture content" of the water-absorbent resin or particulate hydrogel is a value when the mass of a sample is 1 g and the heating temperature is 180°C in the measurement of the "loss on drying" (WSP230.2).

In the present invention, the weight-average particle size (D50) is a value obtained by plotting a ratio R% of particles remaining on a sieve with a predetermined mesh size on a logarithmic probability sheet based on the data of "particle size distribution" (WSP220.2), and reading the mesh size (particle size) at which R is 50.

(Others) In the present invention, the terms "mass" and "weight" are considered synonymous. The expression "from X to Y" that indicates a range means "X or more and Y or less". "X" in the expression "from X to Y" that indicates a range may be a basis of the legality of amendment to "X or more" or "X or less". "Y" in the expression "from X to Y" that indicates a range may be a basis of the legality of amendment to "Y or more" or "Y or less". Further, "%" and "ppm" mean "mass%" and "ppm by mass", respectively, unless otherwise noted. Further, "... acid (salt)" means "... acid and/or a salt thereof", and "(meth)acryl" means "acryl and/or methacryl". The measurements of physical properties and the like are performed at room temperature (from 20 to 25°C) and a relative humidity of 40 to 50% RH unless otherwise specified. When the content, concentration, use amount, or addition amount of a specific component is described, it is a total amount when the specific component contains two or more kinds.

### Method for Producing Water-Absorbent Resin of Present Invention

### Bioethanol

The present invention focuses on bioethanol as a raw material of a water-absorbent resin for the first time, and has a main characteristic of producing a water-absorbent resin from bioethanol which has typically been used mainly as a fuel.

The production amount of bioethanol in the world exceeded 113 billion liters (about 89 million tons) in 2022. Bioethanol corresponds to about 3% of the production amount of petroleum, about 4.6 trillion liters, and is available in a large amount at low cost as a biological raw material. Bioethanol is mainly used for fuels (about 85% for fuels for automobiles and the like), and also used mainly for solvents in industrial applications, and further for beverages, bactericides and the like in food applications. There are cases where bioethanol is used for chemicals such as ethyl ester, but the present invention is characterized in that bioethanol used mainly for fuels is used as a starting material for a water-absorbent resin.

Bioethanol is produced from glucose, sucrose or the like by a known fermentation method, and is produced according to, for example, the following reaction formula using molasses (waste molasses) or the like after separation of a refined sugar as a fermentation raw material.

C₆H₁₂O₆ → 2CH₃CH₂OH + 2CO₂

First, in a first stage, one molecule of glucose is decomposed into two molecules of pyruvic acid by a plurality of glycolytic enzyme. Second and subsequent stages are reactions specific to alcoholic fermentation. One molecule of carbon dioxide is removed from one molecule of pyruvic acid to form acetaldehyde. Thereafter, acetaldehyde is rapidly reduced into ethanol by electrons of the reduced NADH.

An aqueous ethanol solution (having an ethanol concentration of several vol.% to several tens vol.%, particularly from about 5 to 20 vol.%) which is produced by a fermentation method contains a large amount of water, fermentation raw materials (sugars and proteins), and fermented products other than ethanol (organic acids, alcohols, and the like), and thus is purified by distillation, but it forms hydrous ethanol containing a small amount (from about 4 to 10 wt.%) of water even after distillation purification due to an azeotropic phenomenon between ethanol and water. The purity (in particular, concentration) of the hydrous ethanol after distillation can be appropriately selected, but is typically from 90 to 96 wt.%, and in such a hydrous ethanol having a high concentration, most part of impurities is water, and a small amount of impurities are contained.

For further dehydration and further purification of the hydrous ethanol obtained by distillation, the hydrous ethanol is subjected to azeotropic distillation with a hydrophobic organic solvent such as benzene or hexane, or dehydrated with a dehydrating agent such as zeolite or calcium oxide, a reverse osmosis membrane or the like to obtain anhydrous ethanol (usually having a purity of 99.5 vol.% or more) which is commonly distributed. For example, anhydrous ethanol, which has high miscibility with gasoline, is mixed with gasoline and widely used as a fuel.

Both hydrous ethanol and anhydrous ethanol can be used as the bioethanol of the present invention. In a step (i) of producing acetone from bioethanol described below, ethanol and water are reacted according to Formula 1 described below, and therefore, there is no problem even if water is contained in ethanol. It is also one of the characteristics of the present invention that hydrous ethanol, which is cheaper than anhydrous ethanol, can be used as ethanol used as a raw material. On the other hand, the use of anhydrous ethanol is disadvantageous because higher purification cost is required. Further, the hydrophobic organic solvent used for azeotropic distillation during dehydration may remain, which may cause an odor of the water-absorbent resin. Since the flash point of hydrous ethanol is higher than that of anhydrous ethanol (the flash point of anhydrous ethanol is 13.0°C, and the flash point of 50 vol.% ethanol is 22.2°C), the use of hydrous ethanol may be preferred from the viewpoint of the handleability of ethanol.

### Moisture Content of Bioethanol

When hydrous ethanol is used in the present invention, the lower limit of the moisture content of the hydrous ethanol in the present invention may be 3 wt.% or more, 4 wt.% or more, 5 wt.% or more, 6 wt.% or more, 7 wt.% or more, 8 wt.% or more, 10 wt.% or more, or 12 wt.% or more, and the upper limit of the moisture content may be 50 wt.% or less, 40 wt.% or less, 30 wt.% or less, or 20 wt.% or less. The lower limit of the ethanol concentration is appropriately selected within a range of 40 wt.% or more, 50 wt.% or more, 60 wt.% or more, 70 wt.% or more, or 80 wt.% or more. Ethanol having a higher moisture content is preferred because the amount of energy used for distillation purification is small, but the moisture content of the hydrous ethanol is appropriately selected in consideration of impurities, cost or reactivity. For example, by appropriately using hydrous ethanol of which the ethanol concentration is from about 97 to 50 wt.%, from about 96 to 60 wt.%, or from about 96 to 70 wt.%, or ranges from the lower limit to the upper limit, water and ethanol in the hydrous ethanol only, or with water (water vapor) added as necessary, are reacted to produce acetone. For example, when acetone is produced from ethanol according to Formula 1 described below in the step (i) of producing acetone from bioethanol described below, from the weight ratio of 2 mol of ethanol to 1 mol of water (ethanol/water = 92/18) in Formula 1 described below, the theoretical value (degree of reaction of Formula 1 described below being 100%) of the moisture content of the reaction system is about 16 wt.%, and therefore, the ratio between water to be added as necessary and anhydrous or hydrous ethanol and the moisture content of ethanol are selected taking the reaction rate and recovery rate of ethanol, the yield of acetone, energy and the like into consideration as appropriate. An excessively high moisture content (an excessively low ethanol concentration) of the ethanol is not only disadvantageous from an energy point of view in acetone synthesis according to Formula 1 described below, but also disadvantageous in terms of transportation cost for hydrous ethanol containing a large amount of water. Further, removal of fermented products (organic acids and alcohols) other than ethanol may be insufficient during distillation purification from the fermented liquid. The moisture content (vol.% or wt.%) of the hydrous ethanol can be appropriately measured, and can be determined as a characteristic value from, for example, the density (g/ml) (20°C) of the hydrous ethanol.

In the present invention, the moisture content of the hydrous ethanol may be adjusted by distillation conditions for the aqueous ethanol solution (ethanol concentration after fermentation is from several vol.% to several tens vol.%, particularly from about 5 to 20 vol.%), or a predetermined amount of water may be added to the hydrous or anhydrous ethanol after distillation to perform dilution, or the moisture content may be adjusted by mixing a plurality of kinds of anhydrous or hydrous ethanols.

### Minor Components in Bioethanol

When anhydrous or hydrous ethanol is used in the present invention, the ethanol may contain, as minor components other than water, a lower alcohol having 1 or 3 to 5 carbon atoms, such as methanol, 1-propanol, isopropanol, 1-butanol, 2-butanol, 2-methylpropanol, and 2-methyl-1-butanol, and a lower aldehyde or a lower ketone such as acetaldehyde (having 2 carbon atoms) and acetone (having 3 carbon atoms). The content of each of the lower alcohol having 1 or 3 to 5 carbon atoms, acetaldehyde, and acetone in the anhydrous or hydrous ethanol is, for example, 1 wt.% or less, 0.1 wt.% or less, or 0.01 wt.% or less. The contents of these minor components can be measured by, for example, gas chromatography.

In the present invention, acetone or isopropanol may be contained or remain in the ethanol because acetone and isopropanol are produced from the bioethanol, and it is not necessary to remove all of acetone and isopropanol by purification of the ethanol from the viewpoint of the yield of ethanol and cost. In the present invention, bioethanol (and furthermore, hydrous ethanol) containing acetone and/or isopropanol can be used as a preferred starting material. From the viewpoint of cost and performance of the obtained water-absorbent resin, the content of acetone and/or isopropanol in the ethanol is preferably 1 ppm or more, 5 ppm or more, 10 ppm or more, or 20 ppm or more as a total amount of acetone and propanol. More preferably, the content of each of acetone and isopropanol is 1 ppm or more, 5 ppm or more, 10 ppm or more, or 20 ppm or more. The upper limit of the content of acetone and/or isopropanol in the ethanol may be high, but can be selected from 1 wt.% or less, 0.1 wt.% or less, or 0.01 wt.% or less within the above range from the viewpoint of the balance with other impurities. Still more preferably, ethanol containing acetone and isopropanol is used, and the total content of acetone and isopropanol is in the above range. Such ethanol containing acetone and/or isopropanol may be available as crude ethanol.

In the present invention, ethanol produced from a biological raw material can be used, but ethanol produced from a plant raw material is preferably used, and bioethanol produced by fermentation of one or more plant raw materials selected from sugar cane, corn, and sugar beet is more preferably used. One or more selected from sugar cane, corn, and sugar beet may be further pulverized or squeezed before fermentation.

The plant raw material may be a genetically modified plant (typically a genetically modified corn) or a non-genetically modified plant. The present invention involves a route from bioethanol to acetone to isopropanol to propylene to acrylic acid, rather than direct use or consummation of bioethanol, there is no restriction on genetic modification, and it is preferable that a wide range of genetically modified plant raw materials can be used.

Bioethanol can be confirmed by ¹⁴C/¹²C measured by radiocarbon dating. Traceable ethanol is also available. For acetone, isopropanol, propylene, and acrylic acid that are sequentially produced from bioethanol in the present invention, a compound derived from a biological raw material can be confirmed by ¹⁴C/¹²C measured by radiocarbon dating or the record of acquisition and production route.

The bioethanol content can be measured as follows.
1. Ethanol used as a raw material gas is combusted and all converted into carbon dioxide.
2. Carbon dioxide is separated and purified by using a vacuum line.
3. Carbon dioxide produced from ethanol is all reduced by hydrogen using iron as a catalyst, thereby producing graphite.
4. The ratio of the ¹⁴C concentration to the ¹²C concentration
   (¹⁴C/¹²C) in the graphite derived from ethanol is measured using a ¹⁴C-AMS measurement apparatus (available from NEC Corporation, for example).
5. For oxalic acid of the same year produced from the raw material ethanol provided by National Institute of Standards and Technology (NIST) (hereinafter, also referred to as a standard sample), the ratio of the ¹⁴C concentration to the ¹²C concentration (¹⁴C/¹²C) is measured using the same method as in 1 to 4 above.
6. A value produced by dividing the value of ¹⁴C/¹²C of the graphite derived from the raw material ethanol by the value of ¹⁴C/¹²C of the standard sample is multiplied by 100 to obtain the bioethanol content.

### Step (i) of Producing Acetone from Bioethanol

In the method for producing a water-absorbent resin according to the present invention, a step (i) of producing acetone from bioethanol first is essential for production of acrylic acid from bioethanol. A method for producing acetone from ethanol is known, and a known method can be applied for producing acetone from bioethanol. For example, acetone may be synthesized from bioethanol by a method in WO 2022/244797, JP 2012-240913 A, JP 5747326 B, WO 2009/110413, JP 2022-178043 A, or the like. The disclosures of these publications are incorporated by reference in their entirety.

These known Patent Literatures describe the synthesis of acetone from bioethanol, but acetone is generally used as a solvent, and also used as a raw material for methyl methacrylate, and the literatures on the method for producing acetone do not suggest either the production of a water-absorbent resin from ethanol or the production of acrylic acid from ethanol by carrying out steps (i) to (iv). The present invention is characterized in that a water-absorbent resin is produced by carrying out the steps (i) to (iv) among numerous applications of acetone produced from bioethanol, and then carrying out steps (v) to (vii) with bioacrylic acid produced using ethanol as a raw material.

In the present invention, more preferably, acetone is produced according to the following reaction formula (1) of ethanol and water in the step (i). By such a configuration, a water-absorbent resin can be produced more efficiently from a renewable natural raw material.

Formula 1) 2CH₃CH₂OH + H₂O → CH₃COCH₃ + CO₂ + 4H₂

That is, in the present invention, anhydrous or hydrous bioethanol and water (and furthermore, water in hydrous ethanol) are reacted to obtain a mixed gas containing acetone, water vapor, carbon dioxide, and hydrogen. Acetone and isopropanol, which are fermentation by-products, may remain or be contained in the bioethanol.

### Water Used in Reaction

When water (water vapor) is contained in the reaction of Formula 1 in the step (i), the acetone selectivity is improved. The molar ratio of water (water vapor) to ethanol (ethanol gas) (water/ethanol) is preferably from 0.1 to 10, more preferably from 0.5 to 10, still more preferably from 0.5 to 5, and most preferably from 1 to 5, and may be from 1 to 3. When hydrous ethanol is used as bioethanol, the total amount of water contained in hydrous ethanol and brought to the system and water added as necessary is preferably the above molar ratio per 1 of ethanol. As an example, 95 wt.% (about 96 vol.%) of hydrous ethanol contains 0.14 mol of water per 1 mol of ethanol, and in order to set the molar ratio of water to ethanol to 0.1 to 10, water is not added, or 9.9 mol or less of water can be added per 1 mol of ethanol.

Water added as necessary in addition to water in ethanol is not particularly limited, and tap water, industrial water, pure water (RO water, ion-exchanged water, or distilled water), and the like can be used. These waters may be groundwater, river water, or treated water thereof. Water generated in at least one of the steps (i) to (iv) may be reused.

### Catalyst

The catalyst used in the step (i) is not particularly limited, but preferably contains iron, zirconium, and at least one metal (Me) selected from the group consisting of magnesium, calcium, manganese, copper, and zinc. The form of the metal element contained in the catalyst used in the step (i) is not particularly limited, and may be, for example, a metal oxide containing the metal element, a form of a carrier containing the metal element, a form of a carrier supporting the metal element, or a form of a carrier supporting the metal oxide. The metal oxide may be a composite metal oxide. Examples of the composite metal oxide include a spinel type, a perovskite type, a magnetoplumbite type, and a garnet type, and the spinel type is preferred.

The catalyst used in the step (i) preferably contains iron from the viewpoint of catalytic activity. More preferably, the catalyst contains, in addition to iron (Fe), one or more metals (Me) selected from the group consisting of magnesium (Mg), calcium (Ca), manganese (Mn), and zinc (Zn).

The catalyst containing, in addition to iron (Fe), one or more metals (Me) selected from the group consisting of magnesium (Mg), calcium (Ca), manganese (Mn), and zinc (Zn) is preferably an iron composite oxide (which may be referred to as ferrite) represented by General Formula (1) below:

MeO·nFe₂O₃ (1)

(in General Formula (1), Me represents one or more metals selected from the group consisting of Mg, Ca, Mn, and Zn, and n represents a number of 1 to 6).

Specific examples of the iron composite oxide include MgO·Fe₂O₃(MgFe₂O₄) and ZnO·Fe₂O₃(ZnFe₂O₄).

When the catalyst used in the step (i) is a catalyst in which a metal element or a metal oxide is supported on a carrier, examples of the carrier include activated carbon, silica (SiO₂), alumina (Al₂O₃), silica-alumina, zeolite, silica-calcia, zirconia (ZrO₂), ceria (CeO₂), magnesia (MgO), and diatomaceous earth. Among them, one or more selected from activated carbon, silica-calcia, zirconia, ceria, and magnesia are more preferred, and zirconia is particularly preferred. The shape of the carrier is not particularly limited, and examples thereof include a spherical shape, a pellet shape, and a honeycomb shape. The BET specific surface area of the carrier is preferably from 20 to 200 m²/g, and more preferably from 40 to 200 m²/g. The use of a carrier having a high specific surface area is preferred because the catalyst component is easily supported in a dispersed state, and the catalytic activity is enhanced.

The form of the zirconium element contained in the catalyst is not particularly limited, and may be a form of a compound in which zirconium is contained as a single metal, a form in which zirconium is contained as one of the elements of a composite metal oxide formed when zirconium and another metal element are contained, or a form in which zirconium is contained as a carrier. Examples of the compound containing zirconium as a single metal include zirconium oxide (ZrO₂). Examples of the composite metal oxide containing another metal element (Me) include composite metal oxides of zirconium and Sn, Pb, Zn, Cu, Fe, Mn, In, or the like. The carrier is preferably zirconium oxide (ZrO₂), or a composite metal oxide of zirconium, Zn, and Fe, and from the viewpoint of the performance of the catalyst, zirconium oxide (ZrO₂) is more preferred.

The amount of the metal (Me) in the catalyst is preferably from 0.4 to 0.7 mol, more preferably from 0.4 to 0.6 mol, and still more preferably from 0.45 to 0.55 mol, per 1 mol of iron (Fe). When the amount of the metal (Me) is within the above range, good catalytic activity is obtained. The amount of zirconium (Zr) in the catalyst is preferably from 0.01 to 0.5 mol, and more preferably from 0.05 to 0.5 mol, and may be from 0.1 to 0.4 mol, per 1 mol of iron (Fe). When the amount of zirconium is within the above range, the durability of the catalyst can be improved.

The total amount of the metal (Me), iron, and zirconium in the catalyst is preferably from 50 to 100 mass%, and more preferably from 80 to 100 mass%, per 100 mass% of the catalyst.

### Reactor/Reaction Conditions

The reaction in the step (i) may be carried out batchwise or continuously without particular limitation, but is preferably carried out continuously from the viewpoint of productivity. The reaction is preferably a gas phase reaction. Examples of the reaction system of the gas phase reaction include a fixed bed, a moving bed, and a fluidized bed, but the fixed bed system, which is simpler, is preferred.

When the reaction system is a fixed bed system, ethanol gas and water vapor may be mixed and then supplied to an acetone synthesis reactor to come into contact with the catalyst, or ethanol gas and water vapor may be separately supplied to the acetone synthesis reactor to come into contact with the catalyst. Ethanol gas and water vapor may be obtained directly from hydrous ethanol, or water vapor may be added separately. Ethanol gas and water vapor are obtained by heating hydrous or anhydrous ethanol and water, respectively, in a vaporizer. In addition to ethanol gas and water vapor, an inert gas such as nitrogen or helium may be supplied to the acetone synthesis reactor.

The concentration of ethanol gas is preferably from 3 to 66 mol%, and more preferably from 5 to 50 mol%, per 100 mol% in total of gases supplied to the acetone synthesis reactor. With the proportion described above, acetone can be produced with high productivity.

The pressure in the reaction in the step (i) may be reduced pressure, normal pressure, or increased pressure, but is preferably from 0.07 to 2 MPa, and more preferably from 0.1 to 1 MPa. The temperature in the reaction in the step (i) is preferably from 250 to 600°C, more preferably from 300 to 550°C, and still more preferably from 330°C to 500°C. The space velocity of the raw material gas when the reaction is carried out as a gas phase reaction is preferably from 300 to 10000 (1/h), more preferably from 400 to 8000 (1/h), and still more preferably from 500 to 6000 (1/h).

### Purification of Acetone

When the purity of acetone produced in the step (i) is high, acetone can be used as is in the next step (ii) without being purified or isolated, or the step (i) and the step (ii) can be connected. When an acetone-containing mixture produced from the bioethanol contains a gas, the mixture may be separated, by a known gas-liquid separation method, into a gas mainly containing hydrogen, carbon dioxide, or the like and a liquid mixture mainly containing acetone (which may be referred to as gas-liquid separation). The pressure in the gas-liquid separation operation is preferably from 0.1 MPa to 2 MPa, and more preferably from 0.2 MPa to 1 MPa.

Further, an operation of absorbing acetone from a gas mainly containing hydrogen, carbon dioxide, or the like may be performed. The method for absorbing acetone is not particularly limited. The gas may be introduced into an absorption column, where acetone in the gas is absorbed by an absorption liquid supplied from the top of the column, and is recovered as an acetone-containing liquid from the bottom of the column. Water is preferred as the absorption liquid. The water is not particularly limited. Tap water, industrial water, pure water (RO (reverse osmosis) water, ion-exchanged water, or distilled water), and the like can be used. Water used or produced in other steps may be used or reused. The absorption liquid which contains acetone and is obtained from the bottom of the absorption column may be merged with the liquid mixture which mainly contains acetone and is obtained by gas-liquid separation. This makes it possible to improve the recovery rate of acetone.

Next, the acetone-containing mixture, which is a liquid mixture mainly containing acetone, is distilled, whereby purified acetone can be obtained. The distillation can be performed by a known method. Examples of the known distillation method include thin film distillation and rectification. The distillation may be performed continuously or batchwise, but is preferably performed continuously from the viewpoint of productivity.

The purification may be performed only by gas-liquid separation, may be performed by gas-liquid separation and distillation, or may be performed only by distillation, but more preferably includes a gas-liquid separation step and a distillation step in the stated order. This makes it possible to obtain acetone that is more sufficiently purified (which may be referred to as purified acetone).

### Impurities in Bioacetone

For the use of the acetone obtained as described above in the next step (ii), it is preferred that the amounts of aldehydes, alcohols, and ketones (excluding acetone) in the acetone are reduced. It is particularly preferred that the amounts of ethanol and acetaldehyde, which may form acetic acid through the
steps (i) to (iv), are reduced from the viewpoint of the odor (acid odor) of the water-absorbent resin.

### Purity of Bioacetone

The content of acetone contained in the purified acetone obtained in the purification is preferably 90 mass% or more, more preferably 95 mass% or more, and still more preferably 98 mass% or more, per 100 mass% of the purified acetone. For the use of the acetone obtained as described above in the next step (ii), it is preferred that the amounts of aldehydes, alcohols, and ketones (excluding acetone) in the acetone are reduced. It is particularly preferred that the amounts of ethanol and acetaldehyde, which may form acetic acid through steps (ii) to (iv), are reduced. The total content of ethanol and acetaldehyde in the acetone is preferably 20000 ppm or less, more preferably 10000 ppm or less, and still more preferably 5000 ppm or less. The total content of ethanol and acetaldehyde in the acetone is preferably low, but is, for example, 100 ppm or more, 500 ppm or more, or 1000 ppm or more, from the viewpoint of the relationship between the yield of acetone after purification and the purification cost. The total content of ethanol and acetaldehyde in the acetone is, for example, 100 ppm or more and 20000 ppm or less. High-purity acetone with a purity and an amount of impurities in the above ranges as a raw material is subjected to an acetone reduction reaction in the step (ii) below, followed by gas-liquid separation of isopropanol and gas contained in the obtained product, and thus isopropanol having high purity is easily produced. It is also preferred that no special reduction operation is performed when the total content of ethanol and acetaldehyde in the acetone obtained after the step (i) is already in the above range. There is very little or substantially no adverse effect on the water-absorbent resin as long as acetone is obtained with the above purity. Therefore, isopropanol may remain or be contained (for example, 1 ppm or more, or 10 ppm or more) in the obtained acetone from the viewpoint of the purification cost and yield of acetone. The content of these minor components can be measured by, for example, gas chromatography.

### Step (ii) of Producing Isopropanol from Acetone

In the method for producing a water-absorbent resin according to the present invention, a step (ii) of producing isopropanol from acetone is essential for producing acrylic acid from bioethanol. Industrially, isopropanol is produced from acetone by utilizing a cumene method for phenol production, but the method for producing a water-absorbent resin according to the present invention is characterized in that acetone produced from bioethanol in the step (i) is used in the step (ii) in order to solve the problems. A method for producing isopropanol from acetone is known, and a known method can be applied for producing isopropanol (also referred to as 2-propanol or isopropyl alcohol) from acetone produced in the step (i). For example, isopropanol is synthesized from acetone by a method in JP 5197637 B, JP 5300392 B, JP 4321838 B, JP 2762591 B, JP 2723621 B, WO 2022/244797, or the like. The disclosures of these publications are incorporated by reference in their entirety.

These Patent Literatures describe the synthesis of isopropanol from acetone, but isopropanol is generally used as a solvent, and used a starting material for glycerin in chemical products, and the literatures on the method for producing isopropanol do not suggest either the production of a water-absorbent resin from bioethanol or the production of acrylic acid from bioethanol by carrying out the steps (i) to (iv). The present invention is characterized in that a water-absorbent resin is produced by carrying out the steps (i) to (iv) among numerous applications of isopropanol produced from bioethanol, and then carrying out the steps (v) to (vii) with bioacrylic acid produced using ethanol as a raw material.

In the present invention, isopropanol is more preferably produced by hydrogenation of acetone according to Reaction Formula (2) below in the step (ii).

Formula 2) CH₃COCH₃ + H₂ → CH₃CH(OH)CH₃

That is, acetone produced in the step (i) is reacted with hydrogen to obtain a mixed gas containing isopropanol.

### Catalyst

The catalyst used in the step (ii) is not particularly limited, and examples thereof include a Raney catalyst. Examples of the other catalyst include solid catalysts containing a metal element such as Ba, Co, Cr, Cu, Fe, Mn, Ni, Pd, Pt, Zn, Zr, Ru, or Rh. Among them, a solid catalyst containing at least one metal element selected from the group consisting of Pt, Ru, Ni, Fe, and Co is preferred, and at least one solid catalyst selected from the group consisting of a Ru catalyst, a Ni-Pt catalyst, a Ru-Pt catalyst, and a Ni-Ru catalyst is preferably used. With the use of a solid catalyst containing any of these metal elements, the activity inhibition effect of carbon dioxide in the acetone reduction reaction by hydrogen in the step (ii) is suppressed, the acetone hydrogenation efficiently proceeds, and isopropyl alcohol can be produced.

As the catalyst, metal elements can be used in the form of an elemental metal, an alloy, an oxide, or the like. The catalyst may be in the form of a mixture of elemental metals, a mixture of an elemental metal and a metal oxide, a mixture of metal oxides, or a mixed metal oxide.

The catalyst may be a catalyst in which a metal element is supported on a carrier such as activated carbon, silica (SiO₂), alumina (Al₂O₃), titania (TiO₂), zirconia (ZrO₂), ceria (CeO₂), magnesia (MgO), or diatomaceous earth. Among them, silica (SiO₂) and zirconia (ZrO₂) are both preferred as the carrier. The above catalysts may be used alone, or may be used in combination of two or more thereof.

The shape of the catalyst is not particularly limited, and may be any shape such as a ring shape or a spherical shape.

In the step (ii), the catalysts may be used alone, or two or more thereof may be used.

### Reactor/Reaction Conditions

The reaction in the step (ii) may be carried out batchwise or continuously, but is preferably carried out continuously from the viewpoint of productivity. The reaction in the step (ii) is preferably a gas phase reaction. The reaction system of the gas phase reaction is not particularly limited, and examples thereof include a fixed bed, and a fluidized bed, but the fixed bed system, which is simpler, is preferred.

The pressure in the reaction in the step (ii) may be any of reduced pressure, normal pressure, and increased pressure, but is preferably from 0.1 MPa to 2 MPa, and more preferably from 0.1 MPa to 1 MPa.

The temperature in the reaction in the step (ii) is preferably from 20°C to 200°C, and more preferably from 25°C to 150°C. A decreased reaction temperature is advantageous from the viewpoint of equilibrium, but tends to make it difficult for hydrogenation to proceed. On the other hand, when the reaction temperature increases, there is a tendency that the acetone hydrogenation conversion does not increase due to equilibrium constraint, and in addition, hydrogenolysis of acetone and isopropanol occurs, so that the yield decreases.

The space velocity of an introduced material containing acetone when the reaction in the step (ii) is carried out as a gas phase reaction is preferably from 200 to 50000 (1/h), more preferably from 1000 to 20000 (1/h), and still more preferably from 2000 to 10000 (1/h).

### Hydrogen

The hydrogen used may be hydrogen taken out from the gas mainly containing hydrogen, carbon dioxide, and the like, which is obtained in the step (i), unreacted hydrogen in the step (ii) may be reused, or hydrogen may be obtained separately. The amount of hydrogen used is equal to or greater than the equimolar amount of acetone, and preferably ranges from 1 to 10 times, preferably from 1 to 5 times the equimolar amount of acetone from the viewpoint of separation and recovery.

### Separation of Isopropanol

When the obtained isopropanol is a gas-liquid mixture containing isopropanol and a gas, the mixture may be separated, by a known method of gas-liquid separation, into, for example, a gas mainly containing a gas such as hydrogen and a liquid mixture containing isopropanol, followed by recovery of isopropanol. Here, the gas in the present section is a substance that exists as a gas under pressurization and cooling conditions in the gas-liquid separation operation.

In the separation of isopropanol, the pressure in the gas-liquid separation operation is preferably from 0.1 MPa to 2 MPa, and more preferably from 0.2 MPa to 1 MPa.

In the separation of isopropanol, the temperature in the gas-liquid separation operation is preferably from 0°C to 50°C, and more preferably from 5°C to 40°C.

The isopropanol produced by the separation (gas-liquid separation) may be supplied as is to the next step (iii), or may be further purified by distillation as necessary, and then supplied to the next step (iii).

### Purity of Isopropanol Obtained

The purity of isopropanol obtained in the step (ii) is preferably 85 mass% or more, more preferably 90 mass% or more, and still more preferably 93 mass% or more, from the viewpoint of improving the yield and the purity of propylene in the step (iii) described below. In the isopropanol obtained in the step (ii), the concentrations of water and acetone as impurities are each preferably 10000 ppm or less, and more preferably 5000 ppm or less. Since ethanol in isopropanol may form acetic acid through the steps (iii) and (iv), the content of ethanol in the isopropanol obtained in the step (ii) is preferably 20000 ppm or less, more preferably 10000 ppm or less, and still more preferably 5000 ppm or less. The content of ethanol in the isopropanol is preferably low, but is, for example, 100 ppm or more, 500 ppm or more, or 1000 ppm or more, from the viewpoint of the relationship between the yield of acetone after purification and the purification cost. The content of ethanol in the isopropanol is, for example, 100 ppm or more and 20000 ppm or less.

### Step (iii) of Producing Propylene from Isopropanol

In the method for producing a water-absorbent resin according to the present invention, a step (iii) of producing propylene from isopropanol is essential for producing acrylic acid from bioethanol. A method for producing propylene from isopropanol is known, and a known method can be applied for producing propylene from the isopropanol obtained in the step (ii). For example, methods described in JP 2764058 B and JP 2799004 B can be applied. The disclosures of these publications are incorporated by reference in their entirety.

These Patent Literatures describe the synthesis of propylene from isopropanol, but do not describe a biological raw material. Further, propylene is generally used as a raw material for polypropylene (PP), acrylonitrile (ACN), propylene oxide (PO), alcohols, and cumene. The literatures on the method for producing propylene do not suggest either the production of a water-absorbent resin from bioethanol or the production of acrylic acid from bioethanol by carrying out the steps (i) to (iv). The present invention is characterized in that a water-absorbent resin is produced by carrying out the steps (i) to (iv) among numerous applications of propylene produced from bioethanol, and then carrying out the steps (v) to (vii) with bioacrylic acid produced using ethanol as a raw material.

### Catalyst

Examples of the catalyst used in the step (iii) include alumina catalysts, silica-alumina catalysts, magnesia catalysts, zeolite catalysts, and activated clay, as well as catalysts in which any of the catalysts exemplified above serves as a carrier and supports a metal such as titanium oxide (TiO₂), tungsten oxide, or zirconium oxide (ZrO₂). Among them, alumina catalysts are preferred, γ-alumina catalysts are more preferable, and specifically, catalysts in which tungsten oxide is supported on γ-alumina is still more preferable. These may be used alone, or in combination of two or more thereof.

The catalyst may be a catalyst subjected to acid treatment and/or a calcination treatment as necessary on any of those described above. The acid treatment is performed to adjust the acid strength of a catalyst (for example, γ-alumina catalyst) by immersing the catalyst in an acid. Examples of the acid used include aqueous solutions of hydrochloric acid, nitric acid, boric acid, and the like, and carboxylic acids such as acetic acid, formic acid, and oxalic acid.

The form of the catalyst used is not particularly limited as long as it can form a fixed catalyst layer, and examples thereof include a tablet type, a ring type, a spherical type, a cylindrical extrusion type, a three-leaf extrusion type, and a granular type. Among them, the spherical type, the tablet type, and the extrusion type are preferred in that the catalyst strength is high, and the catalyst can be evenly filled in a reaction tube.

When the catalyst is a γ-alumina catalyst, a catalyst is preferably used which has a mean pore diameter, as determined by statistical calculation based on the relationship between the pore diameter and the pore volume, of from 3 to 15 nm, with a standard deviation thereof of from 1 to 4 nm.

### Reactor/Reaction Conditions

The reaction in the step (iii) may be carried out batchwise or continuously, but is preferably carried out continuously from the viewpoint of productivity. The propylene synthesis reaction is preferably carried out as a gas phase reaction. Examples of the reaction system of the gas phase reaction include a fixed bed, a moving bed, and a fluidized bed, but the fixed bed system, which is simpler, is preferred.

The reaction temperature is usually from 150 to 500°C, preferably from 180 to 400°C. The reaction pressure may be any of reduced pressure, normal pressure, or increased pressure, but the reaction system in the catalyst layer is preferably in a gas phase state.

### Gaseous Substances Other than Isopropanol

The raw material mixture used in the step (iii) may contain, in addition to isopropanol, a gaseous substance that is inert to the dehydration reaction of isopropanol for rapidly discharging, from the reaction system, a reaction product containing propylene produced by the dehydration reaction of isopropanol. Examples of such a gaseous substance include nitrogen, helium, and argon. The gaseous substance includes a substance which is a liquid material before being supplied to the reactor, but becomes gaseous under the reaction conditions in the reactor. Examples of such a substance include pentane and hexane.

When the inert gaseous substance is mixed with isopropanol and supplied to the reactor, usually, the amount of the substance used is preferably in a range from 0.01 to 15 mol or from 0.05 to 10 mol per 1 mol of isopropanol. When the amount of the gaseous substance used is excessively large, it may be necessary that a large amount of inert gas is separated from a mixture of propylene and water as a reaction product, and circulated to the reactor, resulting in an economic disadvantage such as an increase in separation cost and cost required for circulation.

### Impurities and Purification of Biopropylene

The product obtained by dehydration reaction of isopropanol (molecular weight of 60.1) has the following approximate composition. Propylene (molecular weight of 42.08): about 70 wt.%, water (molecular weight of 18.0): about 30 wt.%, isopropanol: 1 wt.% or less, acetone: 1 wt.% or less, diisopropyl ether: 1 wt.% or less, and others (impurities originally contained in raw material isopropanol), where acetone and diisopropyl ether are by-products of the dehydration reaction of isopropanol. Here, the expression "about X" (X is a numerical value) means that X × ±10% can be included in addition to X itself.

In the present invention, propylene can be purified. Specifically, when a reaction mixture containing propylene and water as main components is pressurized and/or cooled, oil-water separation occurs, leading to formation of two phases including a propylene layer as the upper layer and a water layer as the lower layer. By the oil-water separation step, a large amount of water produced by the dehydration reaction of isopropanol can be removed from the dehydration reaction product.

The pressure during the pressurization is preferably from 5 to 50 kg/cm² G from the viewpoint of separation and purification cost. The reaction product (in a gaseous form) can be easily liquefied by simply cooling the reaction product as is to 20 to 50°C. The propylene obtained by the separation (gas-liquid separation) may be supplied as is to next step (iv), or may be further purified by distillation as necessary, and then supplied to the next step (iv).

Since the amount of water dissolved in the oil layer is usually as small as 1000 ppm or less, water can be easily separated from propylene in the subsequent distillation purification step. As a result, high-purity propylene free of water can be produced. A propylene fraction flowing out from the distillation column can be allowed to pass in a liquid or gaseous form through a packed bed filled with a commonly used desiccant such as molecular sieve, thereby obtaining propylene that is substantially free of water. As the oil layer after oil-water separation, in addition to propylene, high-purity propylene may be obtained by removing a small amount of impurities by distillation purification.

### Step (iv) of Producing Acrylic Acid from Propylene

In the method for producing a water-absorbent resin according to the present invention, a step (iv) of producing acrylic acid from propylene is essential for producing acrylic acid from bioethanol. A method for producing acrylic acid from propylene is known, and a known method can be applied for producing acrylic acid from the propylene produced in the step (iii). For example, using the methods described in JP 3948837 B, JP 4520637 B, JP 3938646 B, JP 4765165 B, and the like, as appropriate, gas phase oxidation of propylene to acrolein and further to acrylic acid, collection of oxidation products, and purification by distillation and crystallization may be performed. The disclosures of these publications are incorporated by reference in their entirety.

These Patent Literatures do not describe a biological raw material, and do not suggest either production of a water-absorbent resin from bioethanol, or the method for producing water-absorbent resin according to the present invention from bioethanol by carrying out the steps (i) to (iv) and the steps (v) to (vii).

For producing bioacrylic acid by oxidizing biopropylene in the step (iv), a known method for producing acrylic acid by oxidizing propylene produced by cracking naphtha as a fossil raw material, and known production equipment can be directly applied. That is, in the method for producing acrylic acid for use in the present invention, glacial acrylic acid (anhydrous acrylic acid), and furthermore, acrylic acid for a water-absorbent resin, which is used in the present invention can be produced by producing acrylic acid from biopropylene through acrolein with production equipment for acrylic acid (single-stage or multi-stage gas phase oxidation of propylene derived from a fossil raw material), which has been typically operated in the world, and collecting acrylic acid, and further distilling and/or crystallizing acrylic acid.

In the reaction of the step (iv), propylene is oxidized by contacting a molecular oxygen-containing gas such as oxygen or air in the presence of a known catalyst. Usually, the oxidation reaction is carried out in two stages. A catalyst used in the first stage reaction allows production of acrolein by gas phase oxidation of propylene gas, and a catalyst used in the second stage reaction is not particularly limited as long as it allows production of acrylic acid by gas phase oxidation of acrolein gas.

### Catalyst

Examples of the catalyst used in the first stage reaction include solid catalysts containing at least one element selected from Fe, Co, Ni, Mo, Bi, Al, and Si, preferably containing at least one selected from Fe, Mo, and Bi, preferably containing a composite oxide containing at least one selected from Fe, Mo, and Bi, more preferably containing at least one of Mo and Bi, and still more preferably containing Mo and Bi.

Examples of the catalyst used in the second stage reaction include solid catalysts containing at least one element selected from V, Mo, Cu, W, Sb, Al, and Si, and preferably containing at least one selected from Mo, V, and W, more preferably containing at least one of Mo and V, and still more preferably containing Mo and V.

### Reaction Conditions

The acrylic acid synthesis reaction may be carried out batchwise or continuously, but is preferably carried out continuously from the viewpoint of productivity. The reaction temperature in the acrylic acid synthesis reaction is usually in a range of from 200 to 400°C. When the acrylic acid synthesis is performed in two stages, the temperature in the first stage and the temperature in the second stage may be the same or different, and the temperature in the second stage may be set lower than the temperature in the first stage. When the temperature in the first stage and the temperature in the second stage are different, the difference may be, for example, from 40 to 60°C.

### Collection of Acrylic Acid

Usually, a mixed gas obtained by the reaction in the step (iv) is brought into contact with a collection liquid (typically water) for collecting acrylic acid from the mixed gas to obtain an acrylic acid-containing aqueous solution. The mixed gas may contain acrylic acid, a molecular oxygen-containing gas, unreacted components (propylene, and acrolein), and by-products (for example, acetone, acrolein, furfural, and formaldehyde). The liquid for collecting acrylic acid from the mixed gas is typically water as described above, but other liquids can also be used. In this case, the acrylic acid-containing aqueous solution is read as an acrylic acid-containing solution. As the collection liquid for acrylic acid in the mixed gas, at least one of water or an organic solvent is used. As the organic solvent, at least one organic solvent selected from methyl isobutyl ketone, diisopropyl ketone, methyl propyl ketone, methyl isobutyl ketone, methyl-t-butyl ketone, n-propyl acetate, n-butyl acetate, diphenyl ether, diphenyl, and the like is used, water and diphenyl ether are preferably used, and water is more preferably used for the collection liquid.

The water as the collection liquid that absorbs acrylic acid is not particularly limited. Tap water, industrial water, pure water (RO water, ion-exchanged water, and distilled water), and the like can be used. These waters may be groundwater, river water, or treated water thereof. Further, as the water used, water for collecting acrylic acid, or water generated or used in other steps may be used after purification or as it is.

### Purification of Acrylic

Acrylic acid is usually obtained by purifying an aqueous solution containing acrylic acid. The aqueous acrylic acid solution may contain acrylic acid, acetic acid, water, and other impurities (maleic acid, propionic acid, furfural, formaldehyde, and the like).

The method for purifying acrylic acid is not particularly limited. Known methods such as distillation and crystallization can be appropriately utilized. The purification may be only distillation, only crystallization, or a combination of distillation and crystallization. The distillation may be performed only once, or a plurality of operations of distillation may be performed in combination. The crystallization may be performed only once, or a plurality of operations of crystallization may be performed in combination. Further, the distillation and crystallization may be performed continuously or batchwise.

### Impurities in Bioacrylic Acid

In the acrylic acid obtained by oxidation of propylene and purification in the step (iv), the content of propionic acid is preferably 500 ppm or less, 400 ppm or less, or 300 ppm or less, and the content of acetic acid is preferably 1500 ppm or less, 1000 ppm or less, 500 ppm or less, 300 ppm or less, or 200 ppm or less. The content of propionic acid in the acrylic acid is, for example, 100 ppm or more, or 200 ppm or more. The content of propionic acid in the acrylic acid is, for example, 100 ppm or more and 500 ppm or less. The content of acetic acid in the acrylic acid is, for example, 100 ppm or more, or 200 ppm or more. The content of acetic acid in the acrylic acid is, for example, 100 ppm or more and 1500 ppm or less. Since the content of propionic acid (more preferably acetic acid) in the acrylic acid is low, the odor (acid odor) of the obtained water-absorbent resin can be reduced. In addition, the yield of the water-absorbent resin (the ratio of the obtained water-absorbent resin to the acrylic acid used) is improved.

The amount of each of preferably one or more, more preferably two or more, still more preferably three or more, even more preferably four or more, particularly preferably five or more, further more preferably all of the six impurities in the acrylic acid, which are protoanemonin, allyl acrylate, allyl alcohol, aldehyde components (particularly, furfural), maleic acid, and benzoic acid, is from 0 to 20 ppm (on a mass basis; the same applies hereinafter). The amount of each of the impurities is preferably from 0 to 10 ppm, more preferably from 0 to 5 ppm, still more preferably from 0 to 3 ppm, particularly preferably from 0 to 1 ppm, and most preferably N. D. (below the detection limit). Among these impurities, the aldehyde components may increase in the acrylic acid derived from a biological raw material, and it is preferable to perform control to reduce the aldehyde components. As a control method, an aldehyde treating agent (for example, hydrazine) is used, or crystallization is performed. The total amount of protoanemonin, allyl acrylate, allyl alcohol, the aldehyde components, maleic acid, and benzoic acid (with respect to the mass of acrylic acid) is preferably 100 ppm or less, more preferably from 0 to 20 ppm, still more preferably from 0 to 10 ppm, and particularly preferably from 0 to 5 ppm. The term "0 ppm" means N. D. The content of these minor components can be measured by, for example, gas chromatography.

For avoiding solidification of acrylic acid (melting point of 14°C) in winter, the acrylic acid obtained may be made into an aqueous solution (for example, an 80 wt.% acrylic acid aqueous solution) from the viewpoint of handleability as a liquid. However, water in the acrylic acid accelerates the formation of acrylic acid dimers, and increased acrylic acid dimers increase residual monomers in the water-absorbent resin. Thus, the amount of water in the acrylic acid is preferably 2 wt.% or less, more preferably 1 wt.% or less, still more preferably 0.5 wt.% or less, even more preferably 0.3 wt.% or less, particularly preferably 0.1 wt.% or less, and most preferably 0.05 wt.% or less. A small amount of water has little adverse effect on the water-absorbent resin, and the content of water in the acrylic acid is, for example, 10 ppm or more, or 50 ppm or more, from the viewpoint of a balance with purification cost. The content of water in the acrylic acid is, for example, 10 ppm or more and 2 wt.% or less. For the same reason, the amount of acrylic acid dimers in the acrylic acid is preferably 1000 ppm or less, 500 ppm or less, or 200 ppm or less. The amount of acrylic acid dimers in the acrylic acid supplied to the step (v) described below is, for example, 1 ppm or more. The amount of acrylic acid dimers in the acrylic acid is, for example, 1 ppm or more and 1000 ppm or less, 500 ppm or less, or 200 ppm or less, and particularly, 100 ppm or less.

These impurities in the acrylic acid are desirably N. D., but it is difficult to completely remove them even by the steps (i) to (iv). Therefore, acrylic acid containing a certain amount of impurities may be used in the step (v), and at least a part of the impurities in the acrylic acid (for example, acetic acid and propionic acid in the acrylic acid) may be removed in the step (v) and/or the step (vi) by heating in the production process of the water-absorbent resin. For example, at least a part of one or more selected from acrylic acid, acetic acid, and propionic acid that remain in the reaction system may be removed by heat of polymerization which is generated in the step (v).

The acrylic acid produced from bioethanol by carrying out the steps (i) to (iv) in the present invention, in which the amount of organic acids that tend to increase in known bioacrylic acid (and furthermore, acetic acid and propionic acid, in particular, propionic acid) can be reduced as described above, can thus be preferably used for a water-absorbent resin. In addition, the acrylic acid contains few other impurities. A water-absorbent resin that is equivalent or superior to acrylic acid derived from a fossil raw material can be provided as described below.

Accordingly, an aspect of the present invention is a method using, as a monomer of a water-absorbent resin, bioacrylic acid derived from a biological raw material and produced by the following steps (i) to (iv):
(i) producing acetone from bioethanol;
(ii) producing isopropanol from the acetone;
(iii) producing propylene from the isopropanol; and
(iv) producing acrylic acid from the propylene.

### Polymerization Inhibitor

The obtained acrylic acid may contain a polymerization inhibitor. For example, from 1 to 300 ppm, from 10 to 200 ppm, or from 20 to 80 ppm of a polymerization inhibitor, particularly, p-methoxyphenol, is contained.

### Step (v) of Producing Polyacrylic Acid (Salt) by Polymerization of Aqueous Monomer Solution Containing Acrylic Acid

### Monomer and Acrylic Acid

In the present invention, the acrylic acid obtained in step (iv) is essentially used as a monomer of a water-absorbent resin. The water-absorbent resin of the present invention is a crosslinked polymer produced by crosslinking polymerization of a monomer containing acrylic acid and/or a salt thereof (referred to as "acrylic acid (salt)") as a main component, for example, polyacrylic acid and/or a salt thereof (referred to as "polyacrylic acid (salt)") containing a graft component as necessary. The proportion of acrylic acid is preferably from 50 to 100 mol%, more preferably from 70 to 100 mol%, and particularly preferably from 90 to 100 mol% relative to the total amount of monomers, from the viewpoint of the performance of the water-absorbent resin.

In the present invention, in addition to the acrylic acid obtained in the step (iv), additional acrylic acid may be used in combination, and as the acrylic acid used in combination, acrylic acid from fossil raw materials, other bioacrylic acid produced from a biological raw material other than bioethanol (for example, bioacrylic acids of Patent Literatures 1 to 14), or acrylic acid from known fossil raw materials can be used in combination. The combination use ratio can be appropriately determined, but when the acrylic acid obtained in the step (iv) and additional acrylic acid are used in combination, the use ratio of the acrylic acid obtained in the step (iv) is preferably as high as possible from the viewpoint of performance, Sustainablity and Renewablity, and the amount of the acrylic acid obtained in the steps (i) to (iv) is preferably 1 mol% or more, more preferably 5 mol% or more, more preferably 10 mol% or more, more preferably 20 mol% or more, more preferably 30 mol% or more, more preferably 40 mol% or more, more preferably 50 mol% or more, more preferably 60 mol% or more, and most preferably 70 mol% or more with respect to the total amount of acrylic acid. The amount of the acrylic acid obtained in the steps (i) to (iv) may be 80 mol% or more, 90 mol% or more, or 95 mol% or more with respect to the total amount of acrylic acid. The upper limit may be less than 100 mol%, 95 mol% or less, or 90 mol% or less depending on the production capacity for the acrylic acid obtained in the step (iv). The use ratio of the acrylic acid obtained in the step (iv) is, for example, 1 mol% or more and 100 mol% or less. The use ratio of the acrylic acid obtained in the step (iv) is, for example, 1 mol% or more and less than 100 mol%. Examples of the method of using the acrylic acid obtained in the step (iv) and additional acrylic acid in combination in the present invention include mixing the acrylic acid obtained in the step (iv) and additional acrylic acid (acrylic acid derived from a fossil raw material).

For preventing an increase of residual monomers in the water-absorbent resin, the acrylic acid obtained in the step (iv) is preferably used in the step (v) in a short time after purification, and the time between the step (iv) and the step (v) (including transportation and storage, in particular) is preferably within 10 days, preferably within 5 days, within 2 days, or within 1 day. Preferably, the acrylic acid until use in the step (v) is stored at room temperature or below (preferably 35°C or lower, or from 30°C to the melting point or above), and further stored and transported in an oxygen or air atmosphere.

The neutralization percentage of acid groups of polyacrylic acid and the like in the water-absorbent resin of the present invention is preferably 10 mol% or more, more preferably 40 mol% or more, still more preferably 50 mol% or more, and particularly preferably 60 mol% or more, from the viewpoint of water absorption performance. The neutralization percentage of acid groups of polyacrylic acid and the like in the water-absorbent resin of the present invention is preferably 90 mol% or less, more preferably 85 mol% or less, still more preferably 80 mol% or less, and particularly preferably 75 mol% or less, from the viewpoint of water absorption performance. The neutralization percentage of acid groups of polyacrylic acid and the like in the water-absorbent resin of the present invention is, for example, 10 mol% or more and 90 mol% or less from the viewpoint of water absorption performance. The neutralization may be performed on the monomer, on the hydrogel after polymerization, or on both. Examples of the neutralized salt include alkali metal salts such as sodium, potassium, and lithium, ammonium salts, and amine salts.

The monomer for producing the water-absorbent resin in the present invention may be substantially only acrylic acid (salt). The water-absorbent resin may be produced by using another unsaturated monomer in combination with acrylic acid (salt) (the amount of the other unsaturated monomer is, for example, from 0 to 50 mol%, more than 0 mol% and 48 mol% or less, or from 5 to 45 mol% when the total amount of monomers is 100 mol%). The monomer other than acrylic acid (salt) (other unsaturated monomer) is not particularly limited. Specific examples thereof include methacrylic acid, maleic acid, itaconic acid, 2-(meth)acrylamide-2-methylpropanesulfonic acid, and (meth)acrylamide. These other monomers may be used alone, or in the mixture of two or more of them appropriately. Among them, itaconic acid is produced by a fermentation method, and thus contributes to the use of a biological raw material. Therefore, itaconic acid is preferred when a monomer is used in combination.

Specific examples of the internal crosslinking agent include N,N'-methylenebis(meth)acrylamide, (poly)ethylene glycol di(meth)acrylate, (poly)propylene glycol di(meth)acrylate, trimethylolpropane tri(meth)acrylate, glycerin tri(meth)acrylate, glycerin acrylate methacrylate, ethylene oxide-modified trimethylolpropane tri(meth)acrylate, pentaerythritol hexa(meth)acrylate, triallyl cyanurate, triallyl isocyanurate, triallyl phosphate, triallylamine, poly(meth)allyloxyalkanes, (poly)ethylene glycol diglycidyl ether, glycerol diglycidyl ether, ethylene glycol, polyethylene glycol, propylene glycol, glycerin, pentaerythritol, ethylenediamine, ethylene carbonate, propylene carbonate, polyethyleneimine, and glycidyl (meth)acrylate. According to a preferred embodiment, the average number of polyethylene glycol units of the (poly)ethylene glycol di(meth)acrylate is from 7 to 11. The amount of the internal crosslinking agent used preferably ranges from 0.001 to 2 mol%, more preferably from 0.005 to 0.5 mol%, still more preferably from 0.01 to 0.2 mol%, particularly preferably from 0.03 to 0.15 mol% with respect to the amount of the monomer (excluding the crosslinking agent) from the viewpoint of physical properties.

In the polymerization, 0 to 50 wt.% (with respect to the amount of monomers) of hydrophilic polymers such as starch/cellulose, derivatives of starch/cellulose, polyvinyl alcohol, linear polyacrylic acid (salt), and polyacrylic acid (salt)-crosslinked products (in particular, fine powder of a water-absorbent resin), and 0 to 10 wt.%, or 0 to 1 wt.% (with respect to the amount of monomers) of various foaming agents such as carbonic acid salts (hydrogen carbonate), carbon dioxide, azo compounds and inert organic solvents; various surfactants; chelating agents; chain transfer agents such as hypophosphorous acid (salt); and the like may be added to the reaction system. Preferably, a natural polymer is used in a small amount (for example, 10 wt.% or less) even if necessary, or is not used because it has an adverse effect on the heat resistance and performance of the water-absorbent resin as described above.

According to a preferred embodiment, the chelating agent is amino polycarboxylic acid or amino polyphosphoric acid. As the amino polycarboxylic acid, a compound having 2 to 100, 3 to 20, 4 to 10, or 5 to 8 carboxyl groups can be preferably used. Specific examples thereof include iminodiacetic acid, hydroxyethyl iminodiacetic acid, nitrilotriacetic acid, nitrilotripropionic acid, ethylenediamine tetraacetic acid, diethylenetriamine pentaacetic acid, triethylenetetraamine hexaacetic acid, trans-1,2-diaminocyclohexane tetraacetic acid, N,N-bis(2-hydroxyethyl)glycine, diaminopropanol tetraacetic acid, ethylenediamine dipropionic acid, N-hydroxyethylethylene diamine triacetic acid, glycol etherdiamine tetraacetic acid, diaminopropane tetraacetic acid, N,N'-bis(2-hydroxybenzyl)ethylenediamine-N,N'-diacetic acid, 1,6-hexamethylenediamine-N,N,N',N'-tetraacetic acid, and salts thereof. As the salt, a sodium salt is preferred. Examples of the amino polyphosphoric acid include ethylenediamine-N,N'-di(methylenephosphinic acid), ethylenediamine tetra(methylenephosphinic acid), nitriloacetic acid-di(methylenephosphinic acid), nitrilodiacetic acid-(methylenephosphinic acid), nitriloacetic acid-β-propionic acid-methylenephosphonic acid, nitrilotris(methylenephosphonic acid), cyclohexanediamine tetra(methylenephosphonic acid), ethylenediamine-N,N'-diacetic acid-N,N'-di(methylenephosphonic acid), ethylenediamine-N,N'-di(methylenephosphonic acid), ethylenediamine tetra(methylenephosphonic acid), polymethylenediamine tetra(methylenephosphonic acid), diethylenetriamine penta(methylenephosphonic acid), 1-hydroxyethylidenediphosphonic acid, and salts thereof. The use of a chelating agent (preferably 1 ppm or more, or 10 ppm or more, with respect to the amount of the monomer, with an upper limit being, for example, 1 wt.% or less, or 0.5 wt.% or less) is preferred because the polymerization of the acrylic acid is further stabilized, and a water-absorbent resin having better performances can be obtained. Such a chelating agent may be added in the step (v) and/or subsequent steps to improve performance as described below.

The monomer may be bulk-polymerized (solventless polymerization), but is preferably polymerized as an aqueous solution from the viewpoint of water absorption performance. The total concentration of the monomer component in the aqueous monomer solution is preferably 10 wt.% or more, more preferably 20 wt.% or more, and still more preferably 30 wt.% or more, from the viewpoint of the physical properties of the water-absorbent resin. The concentration is preferably 80 wt.% or less, more preferably 75 wt.% or less, and still more preferably 70 wt.% or less. The total concentration of the monomer component in the aqueous monomer solution is, for example, 10 wt.% or more and 80 wt.% or less. For accelerating polymerization, the dissolved oxygen of the aqueous monomer solution may be reduced (preferably to 5 ppm or less, or 2 ppm or less) by heating or introduction of an inert gas during the polymerization.

### Polymerization Method

The type of polymerization applied to an embodiment of the present invention is not particularly limited, but from the viewpoint of water absorption properties, ease of polymerization control, and the like, preferred examples thereof include spray droplet polymerization, aqueous solution polymerization, reverse phase suspension polymerization, droplet polymerization, bulk polymerization, and precipitation polymerization. Aqueous solution polymerization or reverse phase suspension polymerization is more preferably selected, aqueous solution polymerization is more preferably selected, and continuous aqueous solution polymerization is still more preferably selected. Continuous aqueous solution polymerization is particularly preferable, and continuous belt polymerization or continuous kneader polymerization is applied. The polymerization may be neutralization polymerization of the monomer, or may be acid polymerization of the monomer, followed by post-neutralization.

Examples of the typical method for neutralization after acid polymerization include, but are not limited to, those exemplified in JP 10-101735 A, JP 01-103606 A, JP 62-054751 A, JP 2002-527547 A, JP 03-174414 A, and the like. Examples of the typical reverse phase suspension polymerization method include, but are not limited to, those exemplified in JP 57-158209 A, JP 61-087702 A, JP 03-227301 A, JP 11-005808 A, WO 2004/083284, WO 2009/025235, WO 13/018571, WO 2022/265459, WO 2022/265459, and the like. The disclosures of these publications are incorporated by reference in their entirety.

The polymerization initiator used in an embodiment of the present invention is appropriately selected according to the type of monomer polymerized, and the polymerization type, and one or more polymerization initiators can be selected from those used in production of a usual water-absorbent resin, and used. Therefore, the polymerization initiator is not particularly limited.

Examples of the polymerization initiator include thermally decomposable polymerization initiators, photo-decomposable polymerization initiators, or redox-based polymerization initiators that use a reducing agent in combination to accelerate the decomposition of these polymerization initiators. Specifically, one or more of the polymerization initiators disclosed in US 7265190 B are used. Note that from the viewpoint of handleability of the polymerization initiator and physical properties of the water-absorbent resin, a peroxide or an azo compound is preferably used, a peroxide is more preferably used, and a persulfate is still more preferably used. The peroxide is preferably selected from potassium persulfate, ammonium persulfate, sodium persulfate, t-butyl hydroperoxide, and hydrogen peroxide. The temperature at the start of polymerization is preferably, for example, about from 50 to 100°C.

The amount of the polymerization initiator used is preferably 0.001 mol% or more, more preferably 0.01 mol% or more, and preferably 1 mol% or less, more preferably 0.5 mol% or less, still more preferably 0.1 mol% or less, with respect to the total number of moles of the monomer excluding the internal crosslinking agent. The amount of the reducing agent used is preferably from 0.0001 to 0.02 mol% with respect to the total number of moles of the monomer excluding the internal crosslinking agent.

Instead of the polymerization initiator, the polymerization reaction may be performed by irradiating active energy rays such as radiation, an electron beam, and an ultraviolet ray, or these active energy rays and the polymerization initiator may be used in combination.

The reaction temperature in the polymerization reaction is not particularly limited, but the temperature range from the minimum temperature to the maximum temperature (peak temperature) in the polymerization reaction is preferably a range from 15°C to 130°C, and more preferably a range from 20°C to 120°C. The reaction time and the polymerization pressure are not particularly limited, and may be appropriately set according to the types of the monomer and the polymerization initiator, the reaction temperature, and the like. The polymerization conversion is usually 95% or more, or 98% or more, in particular, 99% or more. The upper limit of the polymerization conversion is 100%, but extended time of polymerization is avoided in consideration of productivity, and the upper limit of the amount of residual monomers may be about 0.05 wt.%, about 0.1 wt.%, or about 0.5 wt.%.

Further, the polymerization temperature and pressure can be appropriately selected, and can be selected such that the range from the minimum temperature to the maximum temperature is a range from 20°C to the boiling point, from 50°C to the boiling point, or from 70°C to the boiling point.

In the present invention, high-purity acrylic acid suitable for a water-absorbent resin is produced from bioethanol by carrying out the steps (i) to (iv), and further, the maximum polymerization temperature in the step (v) is preferably a temperature at which water is easily vaporized from the reaction system, for example, 105°C or higher, or 110°C or higher. The upper limit of the maximum polymerization temperature in the step (v) is, for example, 130°C or 120°C. The maximum polymerization temperature in the step (v) is, for example, 105°C or higher and 130°C or lower. In this way, during polymerization (step (v)), at least a part of impurities (for example, acetic acid and propionic acid) in the acrylic acid can be further vaporized and removed during polymerization as water is vaporized. Here, the expression "at least a part" means, for example, 1 wt.% or more, 5 wt.% or more, or 10 wt.% or more of impurities. By setting the maximum polymerization temperature in the step (v) to a temperature at which water is easily vaporized from the reaction system, it is not necessary to excessively purify impurities in the acrylic acid in the step (iv). Here, from the viewpoint of performance and impurity removal, the moisture reduction percentage (reduction percentage of a moisture content (wt.%) of the hydrogel obtained with respect to the amount (wt.%) of water in the monomer) due to vaporization of water during polymerization is preferably from 1 to 20 wt.%, more preferably from 2 to 15 wt.%, and still more preferably from 3 to 10 wt.%. In the present invention, since acrylic acid having a high purity that is equivalent to or higher than that of acrylic acid derived from a fossil raw material is obtained as compared to known acrylic acid, excessive purification of the obtained acrylic acid is not necessary, and the amount of impurities that are further removed in at least one of the step (iv), step (v), or step (vi) is small. For example, when the impurities in the acrylic acid are removed in at least one of the step (iv), step (v), or step (vi), the removal amount is, for example, 0.1 wt.% or less, or 0.01 wt.% or less, with respect to the mass of the acrylic acid. As a result, the yields of acrylic acid and the water-absorbent resin are improved. The moisture content of the obtained hydrogel may be adjusted by evaporating part of water during polymerization. The moisture content here is preferably in the range of the amount of water of the monomer, for example, about from 20 to 80 wt.%, or about from 30 to 75 wt.%, from the viewpoint of performance.

### Optional Aging Step

An aging step may be carried out as an optional step that follows the polymerization step. In the aging step, the hydrogel after polymerization may be taken out from the polymerization machine, followed by storage of the hydrogel with the above polymerization conversion under heating, preferably at a temperature of 40 to 100°C or 50 to 90°C (for example, for 1 minute to 5 hours), thereby increasing the polymerization conversion and the molecular weight.

### Optional Gel Grinding Step

The gel grinding step is a step of grinding a gel in which the hydrogel (crosslinked hydrogel polymer) obtained in the polymerization step is kneaded or ground to obtain a particulate hydrogel that is micronized to a predetermined size. In the step, for example, the hydrogel is ground with a kneader, a screw extruder such as a meat chopper, or a gel grinding apparatus such as a cutter mill (this is also referred to as gel grinding) to obtain a hydrogel in the form of particles (hereinafter, also referred to as "particulate hydrogel"). When the polymerization step is kneader polymerization, the polymerization step and the gel grinding step are performed simultaneously. When a particulate hydrogel is directly produced in the polymerization process, for example, in gas phase polymerization or reverse phase suspension polymerization, the gel grinding step is not performed in some cases. A step of cutting the hydrogel to an appropriate size may be optionally provided before the gel grinding step. In the gel grinding step, additives used for the polymerization and various additives described below may be mixed with the hydrogel to improve the performance of the water-absorbent resin.

For uniform and efficient drying (and further, removal of impurities in the acrylic acid during drying), the average particle size of the particulate hydrogel may be 5 mm or less, or 2 mm or less, in particular, 1 mm or less. The moisture content of the particulate hydrogel is preferably 30 wt.% or more, and more preferably 45 wt.% or more. The moisture content of the particulate hydrogel is preferably 70 wt.% or less, and more preferably 55 wt.% or less. The moisture content of the particulate hydrogel may be, for example, 30 wt.% or more and 70 wt.% or less, 30 wt.% or more and 55 wt.% or less, or 45 wt.% or more and 55 wt.% or less.

### Optional Step of Recycling Acrylic Acid

In the polymerization step (step (v)) and a drying step (step (vi)) described below, acrylic acid (boiling point of 141°C) may vaporize. The vaporized acrylic acid may be discarded, but from an environmental point of view, and further, from the viewpoint of CO₂ reduction and carbon neutrality, it is preferred that the vaporized acrylic acid is, for example, collected, and recycled. Acrylic acid may be collected by, for example, a method of using water or alkaline water, or cooling, and examples of the method for recycling acrylic acid in the present invention include use of the collected bioacrylic acid or an aqueous solution (or an alkaline aqueous solution) thereof for polymerization in the step (v). The amount of acrylic acid recycled is appropriately determined, but is, for example, from 0 to 20%, or from 0.01 to 10%, with respect to the acrylic acid used for polymerization.

### Step (vi) of Drying Polyacrylic Acid (Salt)

The crosslinked hydrogel polymer obtained in the step (v) can be adjusted to an intended moisture content by the drying step. The step (v) and step (vi) may be all carried out by evaporating part of water in the crosslinked hydrogel polymer with heat of polymerization in the step (v) to continuously perform polymerization and drying, but from the viewpoint of performance, it is preferred that the step (vi) of drying after the completion of the step (v) is separately provided. The preferred moisture content (%) at the start of drying in the step (vi) is in the range described above. That is, the moisture content of the particulate hydrogel at the start of drying may be, for example, from 30 to 70 wt.% or from 45 to 55 wt.%. From the viewpoint of reducing coloration, reducing residual monomers, and removing impurities in acrylic acid, drying is started (charging into a dryer) preferably within 2 hours, more preferably within 1 hour, and may be started (charging into a dryer) within 0.5 hours or within 0.2 hours, after the completion of polymerization (after discharge from a polymerization machine, and after discharge from a gel grinding apparatus when the gel grinding step has been carried out). The drying is performed in a temperature range usually from 60 to 250°C, preferably from 100°C to 220°C, more preferably from 120 to 200°C, and even more preferably from 150 to 190°C. The drying time is preferably about 0.1 to 5 hours. The temperature, air volume, and dew point during drying may be constant or may be changed in multiple stages. As the drying method, one or two of azeotropic dehydration in a hydrophobic organic solvent, hot air drying (in particular, ventilation band drying), stirring drying in a rotary stirring vessel (for example, a steam tube dryer or a rotary kiln), stirring drying in a heat transfer dryer including a stirring blade (for example, a paddle dryer), and fluidized drying on a fluidized bed are preferably used. When a plurality of drying methods are used in combination, a step of pulverizing a dried product (semi-dried product) may be provided between the drying methods to accelerate drying, or a part of undried products may be removed.

According to an embodiment, the drying time depends on the surface area and moisture content of the polymer, the type of dryer, and the like, and is selected so as to achieve an intended moisture content, but from the viewpoint of physical properties such as reduction of residual monomers and removal of impurities in the acrylic acid, hot air drying is performed preferably for 0.1 to 5 hours preferably with hot air containing water vapor and having a dew point of 50 to 100°C, and more preferably with hot air containing water vapor and having a dew point of 60 to 90°C. The moisture content of the water-absorbent resin in the present invention (defined as the amount of water contained in the water-absorbent resin/measured as a loss on drying at 180°C for 3 hours) is not particularly limited, but from the viewpoint of the physical properties of the obtained water-absorbent resin product, the water-absorbent resin is preferably a powder having fluidity even at room temperature, and is in a powder form with a moisture content of more preferably from 0.2 to 30 wt.%, still more preferably from 0.3 to 15 wt.%, and particularly preferably from 0.5 to 10 wt.%. A surface-crosslinking step (vii) described below may be carried out after the completion of drying, or drying may be performed simultaneously with surface-crosslinking at the start of or at the same time as the drying step (vi) or during the drying step (vi) (for example, at a moisture content of 10 to 40 wt.%, or 15 to 30 wt.%). The drying step (vi) may be partially or entirely performed by removing water with heat of polymerization in the polymerization step (v), but it is preferred that the drying step (vi) is separately provided after the polymerization step (v), and it is more preferred that the surface-crosslinking step (vii) is separately provided after the drying step (vi).

In the drying, the oxidation heat in the step (iv) of producing acrylic acid from propylene is preferably used as heat for heating the hot air or the heat transfer portion. Preferably, a heat medium heated with the oxidation heat, typically water vapor, is supplied to the dryer to heat the hot air and the heat transfer portion. When the heat medium used in the drying still has heat, the heat medium may be reheated and reused as a heat medium for drying, or used for heating or maintaining the temperature of the apparatus in the step (v) and subsequent steps, as necessary. The heat medium can also be distributed among the drying and the step (v) and subsequent steps and used.

### Removal of Acrylic Acid Impurities in Drying Step

In the present invention, high-purity acrylic acid suitable for a water-absorbent resin is produced from bioethanol by carrying out the steps (i) to (iv), and further, preferably by performing the drying at a high temperature and a high dew point (for example, from 120 to 200°C or from 150 to 190°C and a dew point of 50 to 100°C) in the step (vi), at least part (1 wt.% or more, or 5 wt.% or more) of impurities (for example, acetic acid (boiling point of 118°C) and propionic acid (boiling point of 141°C)) in the acrylic acid can be vaporized and removed during drying. Therefore, it is not necessary to excessively purify the impurities in the bioacrylic acid in the step (iv).

In the present invention, since bioacrylic acid with a high purity that is equivalent to or higher than that of acrylic acid derived from a fossil raw material is produced as compared to known bioacrylic acid, excessive purification of the bioacrylic acid is not necessary. The amount of impurities that are further removed in the steps (iv) to (vi) is small. For example, the amount of the impurities removed is 0.1 wt.% or less, 0.05 wt.% or less, or 0.01 wt.% or less, with respect to the bioacrylic acid. As a result, the amount of CO₂ is reduced, and the yields of acrylic acid and the obtained water-absorbent resin are improved.

In some applications of the water-absorbent resin, a final product can be obtained by the steps up to the step (vi) of drying the polyacrylic acid and/or a salt thereof without carrying out the step (vii) of performing surface-crosslinking on the polyacrylic acid (salt) as described below. In this case, an aspect of the present invention is a method for producing a water-absorbent resin derived from a biological raw material, the method including the following steps (i) to (vi):
(i) producing acetone from bioethanol;
(ii) producing isopropanol from the acetone;
(iii) producing propylene from the isopropanol;
(iv) producing acrylic acid from the propylene;
(v) producing polyacrylic acid and/or a salt thereof by polymerizing an aqueous monomer solution containing the acrylic acid; and
(vi) drying the polyacrylic acid and/or a salt thereof.

### Optional Pulverization Step

The production method according to the present invention may include, as necessary, a pulverization step of pulverizing the crosslinked hydrogel polymer obtained in the polymerization step, with a pulverizer to obtain a particulate water-absorbent resin after drying the crosslinked hydrogel polymer in the drying step. In particular, when aqueous solution polymerization is performed in the polymerization step, it is preferred to include a pulverization step.

### Optional Classification Step Before or After Surface-Crosslinking

In the present invention, it is preferred that classification is further performed by a classification step to adjust the water-absorbent resin to a powder size according to the purpose. The classification step is preferably provided after the drying step, and more preferably after the pulverization step. When the surface-crosslinking step is included, the classification step is preferably provided before the surface-crosslinking step. More preferably, a second classification step is also provided after the surface-crosslinking step.

The particle size of the water-absorbent resin obtained in the present invention varies depending on the application, but for example, when the water-absorbent resin is used for disposable paper diapers, which are a main application of water-absorbent resins, the weight-average particle size (defined by sieve classification) after classification or as a final product preferably ranges from 200 to 700 µm, more preferably ranges from 250 to 600 µm, and particularly preferably ranges from 300 to 500 µm.

The water-absorbent resin obtained in the present invention varies depending on the application, but for example, when the water-absorbent resin is used for disposable paper diapers, which are a main application of water-absorbent resins, the water-absorbent resin preferably contains from 95 to 100 wt.% of water-absorbent resin powder having a particle size of 850 to 150 µm (a material that passes through a 850 µm standard sieve and does not pass through a 150 µm standard sieve/the standard sieve is a JIS product or an equivalent) after classification or as a final product. In the water-absorbent resin obtained in the present invention, the proportion of fine powder (for example, preferably less than 100 µm, more preferably less than 150 µm) is preferably small, specifically less than 5.0 wt.%, or less than 3.0 wt.%, and particularly, less than 1.0 wt.%. For example, the proportion of polyacrylic acid and/or a salt thereof in the form of fine powder in the polyacrylic acid and/or a salt thereof which are subjected to the step (iv) is less than 5.0 wt.%, less than 3.0 wt.%, or less than 1.0 wt.%. In the water-absorbent resin obtained in the present invention, the proportion of coarse particles (for example, preferably substantially not less than 1000 µm in sieve, more preferably not less than 850 µm in sieve) is preferably small, specifically, preferably 5.0 wt.% or less, and more preferably 1.0 wt.% or less. For example, the proportion of polyacrylic acid and/or a salt thereof in the form of coarse particles in the polyacrylic acid and/or a salt thereof which are subjected to the step (iv) is 5.0 wt.% or less, or 1.0 wt.% or less.

### Preferred Fine Powder Recovery Step

The present invention may include a step of recovering fine powder of a water-absorbent resin as a production step which is not described in Patent Literature 1 or the like. The recovery of fine powder can lead to a more carbon-neutral production method. In the fine powder recovery step, the fine powder is removed from the water-absorbent resin before surface-crosslinking and/or the water-absorbent resin after surface-crosslinking, and recycled to a production process for the water-absorbent resin. That is, a part of the water-absorbent resin can be separated in the step (v) and the subsequent steps, and recycled to the step (v) and/or step (vi). The recovered fine powder is preferably recycled in and before the drying step, and recycled to at least one of the polymerization step (step (v)), the gel grinding step, or the drying step (step (vi)). The fine powder to be recycled can be recycled to the production process for the water-absorbent resin as dry powder, and/or as water-swellable gel after being hydrated.

The recovery of fine powder to the polymerization step is described in WO 92/01008, WO 92/020723, WO 10/046267, WO 11/101188, and the like. Fine powder is mixed with a monomer, followed by polymerization. The recovery of fine powder to hydrogel after polymerization is described in JP 03-152104 A, JP 04-227934 A, JP 04-041532 A, and the like, and fine powder or a hydrate thereof is mixed with the hydrogel produced in the polymerization step, followed by drying. The recovery of fine powder in a granulation step is described in EP 0885917 A2, WO 2015/088242 A1, WO 2017/010660 A1, WO 2019/194399 A, and the like, and the granulated fine powder is further dried (generally recycled to the drying step). In the recovery of fine powder, the fine powder is generally recycled in and before the drying step. The disclosures of these publications are incorporated by reference in their entirety.

The fine powder recovered is fine powder removed in the classification step, preferably fine powder of less than 150 µm (defined by a standard sieve), which contains 50 wt.% or more, or 70 wt.% or more, in particular, 90 wt.% or more, of fine powder of less than 150 µm. The amount of fine powder is appropriately determined within a range from 1 to 40 wt.%, 2 to 35 wt.%, or even 5 to 30 wt.% in the water-absorbent resin produced.

### Step (vii) of Performing Surface-Crosslinking on Polyacrylic Acid (Salt)

In the present invention, further it is preferred to perform surface-crosslinking by a surface-crosslinking step (vii). As described above, the surface-crosslinking step (vii) may be carried out after the completion of the drying step (vi), or the drying step (vi) and the surface-crosslinking step (vii) may be simultaneously performed at the start of the drying step (vi) or during the drying step (vi).

The surface-crosslinking is an operation of improving physical properties by making a portion near the surface of the water-absorbent resin have a higher crosslinking density than the inside of the particles. Separately from the internal crosslinking, only the surface or the surface layer is crosslinked by adding various surface-crosslinking agents (a second crosslinking agent with respect to the internal crosslinking agent) to the water-absorbent resin. The present step improves the water absorption performance under pressure which is required for use for disposable paper diapers, which are a main application of the water-absorbent resin. The surface-crosslinking agent is not particularly limited. A crosslinking agent that reacts with a carboxyl group, in particular, a dehydration reaction crosslinking agent, is preferably used.

More specific examples of the dehydration reaction crosslinking agent include polyhydric alcohol compounds such as ethylene glycol, diethylene glycol, triethylene glycol, propylene glycol, 1,3-propanediol, 2-methyl-1,3-propanediol, glycerin, 1,4-butanediol, 1,5-pentanediol and 1,6-hexanediol; amino alcohol compounds such as ethanolamine, diethanolamine and triethanolamine; alkylene carbonate compounds such as 1,3-dioxolan-2-one (ethylene carbonate) and 4-methyl-1,3-dioxolan-2-one; and oxetane compounds and polyvalent oxetane compounds such as 3-methyl-3-oxetanemethanol. Among them, one or more dehydration reaction crosslinking agents selected from polyhydric alcohols, alkylene carbonates, oxazolidinone compounds, and (polyvalent) oxetane compounds are preferred, and polyhydric alcohols or alkylene carbonates are particularly preferred, for maximizing the effects of the present invention.

Examples of the surface-crosslinking agent include, in addition to these dehydration reaction crosslinking agents, non-dehydration reaction crosslinking agents, for example, epoxy compounds such as ethylene glycol diglycidyl ether and γ-glycidoxypropyltrimethoxysilane; polyvalent isocyanate compounds such as 2,4-tolylene diisocyanate; polyvalent oxazoline compounds such as 1,2-ethylenebisoxazoline; silane coupling agents such as γ-aminopropyltrimethoxysilane; polyvalent aziridine compounds such as 2,2-bishydroxymethylbutanol-tris[3-(1-aziridinyl)propionate]; and polyvalent metals such as beryllium, magnesium, calcium, strontium, zinc, aluminum, iron, chromium, manganese, titanium and zirconium.

From the viewpoint of physical properties, the amount of the surface-crosslinking agent used (the total amount in the case of use of two or more surface-crosslinking agents) preferably ranges from 0.001 to 10 parts by mass, more preferably ranges from 0.01 to 8 parts by mass, still more preferably ranges from 0.05 to 5 parts by mass, and most preferably ranges from 0.1 to 2 parts by mass, per 100 parts by mass of the water-absorbent resin (preferably per 100 parts by mass of the water-absorbent resin in a dried form).

In mixing of the surface-crosslinking agent with the water-absorbent resin, water and/or a hydrophilic organic solvent may be used. The amount of water used preferably ranges from 0.1 to 10 parts by mass, more preferably from 0.5 to 8 parts by mass, and still more preferably from 1 to 5 parts by mass, per 100 parts by mass of the water-absorbent resin (preferably 100 parts by mass of the water-absorbent resin in a dried form). Examples of the hydrophilic organic solvent include alcohols such as ethyl alcohol and isopropanol; ketones such as acetone; and ethers such as dioxane, alkoxy (poly)ethylene glycol, and tetrahydrofuran, and the amount of the hydrophilic organic solvent used preferably ranges from 0 to 10 parts by mass, more preferably ranges from 0 to 5 parts by mass, and still more preferably ranges from 0 to 3 parts by mass, per 100 parts by mass of the water-absorbent resin.

When heat treatment is performed, the treatment time is preferably from 1 to 180 minutes, more preferably from 3 to 120 minutes, and particularly preferably from 5 to 100 minutes. The heat treatment temperature (defined by the temperature of the heat medium or the temperature of the material) preferably ranges from 100 to 250°C, more preferably ranges from 140 to 220°C, still more preferably ranges from 150 to 220°C, and particularly preferably ranges from 160 to 220°C. The heat treatment is preferably followed by forced cooling of the surface-crosslinked water-absorbent resin, in particular, to 40 to 100°C, or 50 to 90°C for terminating the reaction.

### Optional Additive Addition Step

In the production method according to the present invention, additives such as an inactive surfactant, an inactive deodorant and inactive inorganic fine particle powder may be added to the surface of the water-absorbent resin before, during or after the surface-crosslinking step, where the term "inactive" means that the additives do not cause surface-crosslinking and do not substantially react with the water-absorbent resin.

In the production method according to the present invention, various additives may be further added to the aqueous monomer solution and/or the polyacrylic acid (salt) in any of the step (v) and subsequent steps. That is, the method may include an addition step for imparting various functions to the water-absorbent resin by adding, for example, a deodorant, an antibacterial agent, a perfume, a foaming agent, a pigment, a dye, hydrophilic short fibers, a plasticizer, a tackifier, a surfactant, a fertilizer, an oxidizing agent, a reducing agent, water, salts, a chelating agent, a bactericide, a hydrophilic polymer such as polyethylene glycol, paraffin, a hydrophobic polymer, a thermoplastic resin such as polyethylene or polypropylene, a thermosetting resin such as a polyester resin or a urea resin, and the like, preferably to the surface of the water-absorbent resin. For example, the chelating agent added in the step (v) of polymerization or after the step (v) can further improve the polymerization of bioacrylic acid obtained in the steps (i) to (iv) and the performance of the water-absorbent resin.

The amount of the additives used preferably ranges from 0 to 30 parts by mass, more preferably ranges from 0 to 10 parts by mass, and still more preferably ranges from 0 to 1 part by mass, per 100 parts by mass of the water-absorbent resin. In the present invention, the term "water-absorbent resin" is used as a generic term for water-absorbent resins even after surface-crosslinking of the water-absorbent resin and/or addition of additives to the water-absorbent resin as long as the water-absorbent resin is a main component and is substantially integrated.

### Optional Granulation Step

Simultaneously with or separately from the surface-crosslinking step, water or an aqueous binder solution may be added to the surface of the water-absorbent resin to granulate the water-absorbent resin, and dust may be reduced. As the binder for the water-absorbent resin, 0.1 to 5 parts by weight of water, a water-soluble polymer, or a polyhydric alcohol or an aqueous solution thereof is used in an amount of about 0.1 to 5 parts by weight.

### Transportation or Storage of Any Intermediate

The method for producing a water-absorbent resin according to the present invention includes a polymerization step, a drying step, and a surface-crosslinking step, and further includes an optional aging step, an optional gel grinding step, an optional pulverization step, an optional classification step, an optional fine powder recovery step, an optional classification step after surface-crosslinking, and an optional additive addition step, and these steps may be carried out in the same apparatus (for example, gel grinding and drying in a polymerization apparatus, or drying and surface-crosslinking in a drying apparatus), or may be carried out in different apparatuses. When the above steps are carried out in different apparatuses, a step of transporting the hydrogel or a dried product thereof is provided between the steps to connect the steps, and here, a step of storing the hydrogel or dried product thereof as an intermediate (for example, an intermediate hopper) may be optionally provided between the steps.

### Optional Foreign Substance Removal Step

The water-absorbent resin produced through the above production process is optionally subjected to a foreign substance removal step. Examples of the foreign substance in the production process for a water-absorbent resin include metal foreign substances such as metal pieces which are fragments from a classification mesh, and colored particles (black particles or brown particles) generated by burning of a part of the water-absorbent resin particles due to overheating in the production process. The metal foreign substances can be removed by an iron remover (magnet), and the colored foreign substances (colored particles of the water-absorbent resin) can be removed by color sorting.

The flux density of the magnetic field lines in the iron remover is preferably 0.05 Wb/m² (500 gauss) or more, more preferably 0.5 Wb/m² or more, and particularly preferably 1.0 Wb/m² or more. A permanent magnet and/or an electromagnet is preferably used, and magnets are more preferably arranged in a lattice pattern, followed by passage of the water-absorbent resin between lattices.

### Final Storage Step

The production method according to the present invention may include a storage step of storing the dried water-absorbent resin in a storage tank. The storage tank used in the storage step is, for example, a silo or a hopper, and preferably includes a unit for heating the inner wall surface of the storage tank. From the viewpoint of the abrasion and the electrification of the water-absorbent resin, a storage tank having a metallic inner surface, for example, an inner surface made of iron or stainless steel is preferred. In addition to the storage of the final product, a storage step (intermediate hopper) may be separately provided between the steps, or the steps may be connected by a buffer hopper and a quantitative supply hopper for continuous production.

### Combined Use of Acetone, Isopropanol, and Propylene Derived From Fossil Raw Materials

As additional bioacrylic acid derived from a fossil raw material or produced from a biological raw material other than bioethanol may be used in combination as acrylic acid used in the step (v), a part of each of the acetone in the step (ii), the isopropanol in the step (iii), and the propylene in the step (iv) may contain at least one of acetone, isopropanol, or propylene derived from a fossil raw material or derived from a biological raw material other than bioethanol as long as at least a part of monomers forming the backbone of the water-absorbent resin contains monomers derived from bioethanol. For example, when crops used as raw materials for bioethanol are not sufficiently produced, or a surplus or by-products of a compound derived from a fossil raw material and generated in production of another compound are treated, at least one of acetone, isopropanol and propylene derived from a fossil raw material or derived from a biological raw material other than bioethanol can be used in combination. From the fact that the amounts of carbon isotopes ¹³C and ¹⁴C vary depending on the type of plant used as a raw material or the difference between a fossil raw material and a non-fossil raw material, water-absorbent resins having various amounts of carbon isotopes ¹³C and ¹⁴C can be produced by combined use of a plurality of raw materials at various ratios. By measuring the amounts of carbon isotopes ¹³C and ¹⁴C, the traceability (identifiability) of the produced water-absorbent resin can be revealed. When at least one or more selected from acetone, isopropanol, propylene, and acrylic acid derived from a fossil raw material and/or derived from a biological raw material other than bioethanol are used in combination, the proportion of the acrylic acid derived from bioethanol in the monomers forming the backbone of the final water-absorbent resin is preferably 1 mol% or more, more preferably 5 mol% or more, more preferably 10 mol% or more, more preferably 20 mol% or more, more preferably 30 mol% or more, more preferably 40 mol% or more, more preferably 50 mol% or more, more preferably 60 mol% or more, and more preferably 70 mol% or more. The proportion may be 80 mol% or more, 85 mol% or more, 90 mol% or more, or 95 mol% or more.

### Method for Connecting Step (i) to Step (vii)

The steps (i) to (vii) may be connected and continuously performed, or may be individually performed. Further, the individual purification of at least one of the acetone, isopropanol, propylene, or acrylic acid described for the steps (i) to (iv) may be omitted. The steps (i) to (vii) may be carried out by the same producer, or part or all of the steps may be carried out by different producers. For example, the following sharing is possible.
Company A: the step (i) of producing acetone from bioethanol
Company B: the step (ii) of producing isopropanol from bioacetone
Company C: the step (iii) of producing propylene from bioisopropanol
Company D: the step (iv) of producing acrylic acid from biopropylene
Company E: the step (v) of producing polyacrylic acid and/or a salt thereof by polymerizing an aqueous monomer solution containing bioacrylic acid
Company F: the step (vi) of drying polyacrylic acid and/or a salt thereof
Company G: the step (vii) of performing surface-crosslinking on polyacrylic acid and/or a salt thereof

The steps (i) to (vii) may be performed at the same location or at different locations. The same location means a positional relationship which enables connection through a pipeline within an industrial site. When the steps are carried out at different locations, a transportation method other than a pipeline, for example, long-distance transportation with a tanker, truck, railway, or the like is used.

As described above, the bioethanol may be anhydrous ethanol, hydrous ethanol, or ethanol containing acetone or isopropanol (crude ethanol, in particular, crude hydrous ethanol).

In the step of producing bioethanol (referred to as a step (0)) before the step (i), fermentation and distillation, and optionally another type of purification are performed to adjust the moisture content and minor components (such as acetone and isopropanol) of ethanol used, as described above. The step (i) of producing acetone from bioethanol and the step (0) of producing ethanol (whose moisture content and minor component content are adjusted as necessary) may be performed at the same location or different locations, and the steps (0) and (i) may be connected through a pipeline described below.

From the viewpoint of the performance of the water-absorbent resin, the steps (v) and (vi) are preferably carried out at the same location, the steps (v) to (vii) are more preferably carried out at the same location, the number of locations for the steps (i) to (vii) is still more preferably at most 4, or at most 3 or 2, and all the steps are particularly preferably carried out at one location.

Further, the step (iv) is an exothermic reaction involving oxidation, and therefore, by carrying out the step (iv) and the steps (v) and (vi) at the same location, heat generated in the step (iv) can be used for heating for polymerization in the step (v), drying in the step (vi), and/or surface-crosslinking in the step (vii), etc., so that CO₂ can be further reduced. Thus, a more environmentally friendly method for producing a water-absorbent resin can be achieved. For example, heat can be supplied as high-pressure steam to at least one of the steps (v), (vi), or (vii) through a pipeline. In addition, since both the dehydration in the step (iii) and the oxidation in the step (iv) are reactions in a gas phase, and in the dehydration reaction of isopropanol in the step (iii), the yield of biopropylene is high, the purification of biopropylene in the step (iii) can be omitted to continuously carry out the steps (iii) and (iv). From the viewpoint of reducing CO₂, it is preferred to continuously carry out the steps (iii) and (iv), and further, the steps (iii) to (vii) at the same location.

On the other hand, acetone is easy to transport because it has a reduced weight as compared to the ethanol consumed, and isopropanol is easy to transport because it is easily handled.

Ethanol (boiling point of 78°C), acetone (boiling point of 56°C), isopropanol (boiling point of 82°C), propylene (boiling point of -47°C) and acrylic acid (boiling point of 141°C) which are used or produced in the steps (i) to (v) are handled as a liquid or a gas, and among them, propylene may be liquefied by cooling or may be handled as a gas, and may be appropriately transported (for example, transported through a pipeline) and stored (for example, stored in a tank having a liquid cooling or circulation mechanism) as a liquid or a gas between the steps. When propylene is liquefied and transported, at the time of use of the liquefied propylene in the step (iv), a cooling and heating medium may be produced by recovering latent heat from the propylene, and used for cooling in the step (iv) and subsequent steps. Since the polyacrylic acid produced from liquid acrylic acid in the step (v) is a gelatinous substance, transportation and storage suitable for the gelatinous substance are selected between the steps (v) and (vi). That is, in the steps (v) to (vii), the product is a gelatinous substance or a solid (in particular, powder), and therefore is appropriately conveyed by various conveyors, pneumatic transportation, or the like.

The transportation between adjacent steps among the steps (i) to (vii) depends on a location and a manufacturer for each step, but for solving the above problems, the transportation of the biological raw material between at least one pair of adjacent steps among the steps (i) to (vii) (preferably between at least one pair of adjacent steps among the steps (i) to (v)) is performed by long-distance transportation over 10 km or more using a tanker, a truck, or a railway, and another transportation is also performed in combination between at least another pair of adjacent steps (preferably between the steps (i) to (iv) and between the steps (v) to (vii)). Such a configuration enables production of a more optimal water-absorbent resin.

For solving the above problems, the transportation of a biological raw material between at least one pair of adjacent steps among the steps (i) to (vii) is performed through a pipeline connecting the steps, and transportation is optionally performed between other pairs of adjacent steps by means other than a pipeline. Such a configuration enables production of a more optimal water-absorbent resin. In the steps (i) to (iv), the product is a liquid or a gas, and therefore when the steps are carried out by one or more companies, the steps are preferably connected through a pipeline, and one or more, or two or more pairs of adjacent steps, in particular, all steps are connected through a pipeline. The length of the pipelines over all the steps is appropriately adjusted to, for example, 100 km or less, or 10 km or less, in particular, 1 km or less.

The step (0) of producing bioethanol and the step of producing bioacetone from bioethanol as described above may also be performed by one or more companies, and transportation between the steps (0) and (i) is performed by the same means as in the steps (i) to (vii), or the steps (0) and (i) are connected through a pipeline.

Further, from the viewpoint of reducing residual monomers in the water-absorbent resin, the steps (iv) and (v) are preferably performed within a certain period of time (particularly including transportation and storage), that is, within 10 days, further within 5 days, within 2 days, or within 1 day, as described for the step (v). Here, the temperature and conditions for storage and transportation of the acrylic acid are as described above.

Part or all of the steps (i) to (vi) may be performed at a plurality of locations and by a plurality of manufacturers, or by methods with different conditions within the scope of the present invention. The phrase "methods with different conditions within the scope of the present invention" means, for example, a case where purified and non-purified raw materials are used in combination in the next step, or a case where raw materials produced using different catalysts are used in combination in the next step.

### Difference from Prior Art

As described above, in the method for producing a water-absorbent resin according to the present invention, a water-absorbent resin having equivalent or higher performance and an equivalent or reduced amount of impurities as compared to a known water-absorbent resin derived from a fossil raw material can be produced at low cost by preparing a water-absorbent resin, for the first time, from inexpensive bioethanol as a starting material for the water-absorbent resin instead of a method using, as a raw material for the water-absorbent resin, a natural polymer poor in performance and heat resistance and instead of a known and typical method for producing acrylic acid derived from a biological raw material (the starting material is glycerin, bionaphtha, lactic acid, 3-hydroxypropionic acid, or bionaphtha based on natural oil and/or fat,), as a method for producing a water-absorbent resin derived from a biological raw material.

Patent Literatures 1 to 20 and Non-Patent Literature 1 described above, which relate to water-absorbent resins, do not suggest the production of a water-absorbent resin from bioethanol by the present steps (i) to (vii). Known techniques, as well as the patent literatures described for the steps (i) to (iv), do not suggest either the production of a water-absorbent resin from bioethanol or the production of acrylic acid from bioethanol by carrying out the steps (i) to (iv). In known methods for producing a water-absorbent resin derived from a biological raw material, such as those described in Patent Literatures 1 to 20, an expensive biological raw material (and further, a biological raw material whose production amount is limited) is required for bioacrylic acid, and the obtained water-absorbent resin is insufficient because of, for example, an increased amount of impurities, in particular, an increased amount of organic acids (in particular, propionic acid, for example), in the acrylic acid even when purification is performed to a greater degree than in the past. Patent Literatures and Non-Patent Literatures described above do not suggest, as a solution to address the present problems, the present method for producing a water-absorbent resin from bioethanol by carrying out the steps (i) to (vii).

### Water-Absorbent Resin of Present Invention

In the present invention, a water-absorbent resin produced by the production method described above is provided.

The water-absorbent resin produced through the production method (production process) has the following target performance.

The target performance varies depending on the water-absorbent resin, and examples thereof include, in particular, centrifuge retention capacity, absorption against pressure, particle size distribution, water absorption speed, liquid permeability, fluidity, color, dust amount, deodorizing performance, and antibacterial performance. Typical performance of water-absorbent resins can be found in the WSP (Worldwide Strategic Partners) standards of EDANA Recommended Test Methods, and examples thereof include, but are not limited to, pH (WSP200.2), residual monomers (WSP210.2), a particle size distribution (WSP220.2), a loss on drying (WSP230.2), FSC (WSP240.2), CRC (WSP241.2), AAP (WSP242.2), PDAUP (WSP243.1), Flow Rate (WSP250.2), bulk specific gravity (WSP260.2), water-soluble content (WSP270.2), absorbed particles (WSP280.2), dust (WSP290.2), as well as non-WSP-defined performance such as liquid permeability (SFC and GBP), coloration (YI/WB), and water absorption speed (Vortex/FSR/DW).

As an example, the water-absorbent resin of the present invention has the following performance.

### Amount of ¹⁴C

The ratio of the biological raw material can be identified by ¹⁴C (radioactive carbon)/¹²C (carbon) in the polyacrylic acid obtained. In a known acrylic acid (salt)-based water-absorbent resin produced from a fossil raw material (in particular, petroleum, or propylene), ¹⁴C/¹²C is less than 1.0 × 10⁻¹⁴. On the other hand, in the water-absorbent resin of the present invention, ¹⁴C/¹²C is preferably 1.0 × 10⁻¹⁴ or more, more preferably 1.0 × 10⁻¹³ or more, still more preferably 5.0 × 10⁻¹³ or more, and particularly preferably 1.0 × 10⁻¹² or more. When the non-fossil raw material accounts for substantially 100 wt.%, the upper limit is 1.25 × 10⁻¹². ¹⁴C/¹²C can be measured by, for example, isotope mass spectrometry, and is described in, for example, US 3885155, US 4427884, US 5438194, and US 5661299. A specific measurement procedure is shown below.
1. An acrylic acid (salt)-based water-absorbent resin is combusted and converted into carbon dioxide.
2. Carbon dioxide is separated and purified by using a vacuum line.
3. Carbon dioxide produced from the acrylic acid (salt)-based water-absorbent resin is reduced with hydrogen using iron as a catalyst, thereby producing graphite.
4. The ratio of the ¹⁴C concentration to the ¹²C concentration
(¹⁴C/¹²C) in the graphite derived from the acrylic acid (salt)-based water-absorbent resin is measured using a ¹⁴C-AMS measurement apparatus.

The amount of ¹⁴C (radioactive carbon) can be adjusted by the use ratio of the biological raw material (in particular, bioethanol).

### Amount of ¹³C

The stable carbon isotope ratio (δ¹³C) measured by accelerator mass spectrometry can be appropriately adjusted within a range from 0 to -40 ‰ (per mil). The stable carbon isotope ratio (δ¹³C) can be adjusted by the type of plant as a raw material, and can be appropriately adjusted by raw material adjustment using a C3 plant of δ¹³C ≤ -20 ‰ (wheat flour, potato, rice, or the like) and a C4 plant of δ¹³C ≥ -20 ‰ (corn or the like). For the measurement method, see Patent Literatures 13 and 14.

### CRC (WSP241.2)

The CRC (centrifuge retention capacity) of the water-absorbent resin obtained in the present invention is preferably 10 [g/g] or more, more preferably 20 [g/g] or more, still more preferably 25 [g/g] or more, and particularly preferably 27 [g/g] or more. The upper limit value of CRC is not particularly limited, but is preferably 50 [g/g] or less, more preferably 45 [g/g] or less, still more preferably 42 [g/g] or less, and most preferably 35 [g/g] or less, from the viewpoint of the balance of other physical properties. The CRC can be appropriately controlled by the amount of a crosslinking agent during polymerization and subsequent surface-crosslinking (secondary crosslinking). The CRC (centrifuge retention capacity) of the water-absorbent resin obtained in the present invention is, for example, 10 [g/g] or more and 50 [g/g] or less.

### AAP (WSP242.2)

For the AAP (absorption against pressure) of the water-absorbent resin obtained in the present invention, AAP does not decrease because the purity of acrylic acid for use in the present invention is equivalent to or higher than that of acrylic acid from a typical fossil raw material. For preventing leakage in disposable diapers, when the polymerization is an example as means for achievement, AAP under a pressure of 2.1 kPa or 4.8 kPa is preferably 17 [g/g] or more, more preferably 20 [g/g] or more, still more preferably 22 [g/g] or more, even more preferably 23 [g/g] or more, and most preferably 24 [g/g] or more. The upper limit value of AAP is not particularly limited, but is preferably 35 [g/g] or less, more preferably 30 [g/g] or less, and still more preferably 28 [g/g] or less, from the viewpoint of the balance with other physical properties. The AAP can be improved (adjusted) by surface-crosslinking, after the drying step (iv), and preferably after particle size control. The value of AAP may change depending on a step performed after the surface-crosslinking step. The AAP (absorption against pressure) of the water-absorbent resin obtained in the present invention is, for example, 17 [g/g] or more and 35 [g/g] or less as the AAP under a pressure of 2.1 kPa or 4.8 kPa.

### Water-Soluble Content (WSP270.2)

For the water-soluble content of the water-absorbent resin obtained in the present invention, the soluble content does not increase because the purity of bioacrylic acid is equivalent to or higher than that of acrylic acid from a typical fossil raw material. For preventing stickiness or the like during use in disposable diapers, which is caused by a liquid elution content, the water-soluble content is preferably 35 wt.% or less, more preferably 25 wt.% or less, still more preferably 15 wt.% or less, even more preferably 10 wt.% or less, even more preferably less than 10 wt.%, and particularly preferably less than 8.3 wt.%. The water-soluble content can be appropriately controlled by controlling the amount of the crosslinking agent during polymerization, and preferably by cleaving chemical bonds under a mechanical action in the subsequent gel grinding. The lower limit of the water-soluble content in the water-absorbent resin obtained in the present invention is, for example, 4.0 wt.% or more.

### SFC (Saline Flow Conductivity)

The SFC (saline flow conductivity) of the water-absorbent resin obtained in the present invention can be improved by surface-crosslinking after the above production method, in particular, after the gel grinding in the present invention, preferably after the particle size control, for preventing leakage in disposable diapers, and when surface-crosslinking until AAP falls within the above range is an example of means for achievement, the flow conductivity (SFC) of a 0.69% aqueous sodium chloride solution, which is a liquid permeability property under pressure, is preferably 10 [× 10⁻⁷·cm³·s·g⁻¹] or more, more preferably 20 [× 10⁻⁷·cm³·s·g⁻¹] or more, still more preferably 30 [× 10⁻⁷·cm³·s·g⁻¹] or more, even more preferably 50 [× 10⁻⁷·cm³·s·g⁻¹] or more, particularly preferably 70 [× 10⁻⁷·cm³·s·g⁻¹] or more, and most preferably 100 [× 10⁻⁷·cm³·s·g⁻¹] or more.

### Residual Monomers (WSP210.2)

The water-absorbent resin obtained in the present invention has an advantage that the amount of residual monomers is small because bioacrylic acid is produced with a high purity. From the viewpoint of safety, when the polymerization is an example as means for achievement, the amount of residual monomers is controlled to be usually 500 ppm or less, preferably less than 500 ppm, more preferably from 0 to 450 ppm, still more preferably from 0 to 400 ppm, particularly preferably from 0 to 300 ppm, and particularly preferably from 0 to 200 ppm. The residual monomer can be appropriately controlled by, for example, a polymerization initiator during polymerization, and conditions for subsequent drying.

### Amount of Impurities Other than Residual Monomers

In the water-absorbent resin obtained in the present invention, the amount of impurities does not increase because the purity of acrylic acid used in the present invention is equivalent to or higher than that of known acrylic acid from a fossil raw material. Further, there are no coloration and odor problems. Typical impurities of the water-absorbent resin other than residual monomers include acetic acid and propionic acid, and the total content thereof in the water-absorbent resin is preferably 1000 ppm or less, 800 ppm or less, 600 ppm or less, 500 ppm or less, 400 ppm or less, 300 ppm or less, or 250 ppm or less. The total content of acetic acid and propionic acid in the water-absorbent resin obtained in the present invention is preferably small, but from the viewpoint of a balance with reduction cost and a possibility that excessive reduction deteriorates performance, the total content may be, for example, 100 ppm or more, or 200 ppm or more. The total content of acetic acid and propionic acid in the water-absorbent resin obtained in the present invention is, for example, 100 ppm or more and 1000 ppm or less, or 100 ppm or more and 250 ppm or less.

The total content of acetic acid, propionic acid and residual monomers (in particular, acrylic acid), which are causes of the acid odor of the water-absorbent resin, is preferably 1500 ppm or less, 1200 ppm or less, 1000 ppm or less, 900 ppm or less, 800 ppm or less, 700 ppm or less, or 685 ppm or less. The total content of acetic acid, propionic acid, and residual monomers (in particular, acrylic acid) in the water-absorbent resin is preferably small, but from the viewpoint of a balance with cost and a possibility that excessive reduction deteriorates performance, the total content may be, for example, 100 ppm or more, or 200 ppm or more.

### FSR (Water Absorption Speed)

For preventing leakage in disposable diapers, when the polymerization is an example of means for achievement, the FSR (water absorption speed) of the water-absorbent resin obtained in the present invention is usually 0.20 [g/(g·s)] or more, preferably 0.25 [g/(g·s)] or more, more preferably 0.30 [g/(g·s)] or more, still more preferably 0.35 [g/(g·s)] or more, particularly preferably 0.40 [g/(g·s)] or more, and most preferably 0.45 [g/(g·s)] or more. The upper limit value of FSR is 1.00 [g/(g·s)] or less. The method for measuring FSR is defined in WO 2009/016055. The FSR can be adjusted by the production method of the present invention and the particle size control after drying.

### Particle Size Distribution

The water-absorbent resin of the present invention may be in the form of a sheet or a fiber, but is preferably adjusted to the particle size (preferably from 850 to 150 µm, etc.) described above (Optional Classification Step Before or After Surface-Crosslinking).

### Coloration

The water-absorbent resin is white with a YI (yellow index) of 20 or less, 15 or less, or 10 or less.

### Performance of Representative Water-Absorbent Resin

The present invention provides, as an example, a water-absorbent resin derived from a biological raw material, which satisfies the following performance, and further satisfies the above performance and which has equivalent performance and an equivalent or reduced amount of impurities as compared to a water-absorbent resin derived from a 100% fossil raw material.
CRC = 10 to 50 g/g
AAP 2.1 kPa ≥ 17 g/g
AAP 4.81 kPa ≥ 17 g/g
Water-soluble content ≤ 35%
Residual monomers ≤ 500 ppm
FSR ≥ 0.20 g/g/sec.

Preferably, the water-absorbent resin has the following performance.

CRC = more than 27.5 g/g and 50 g/g or less, AAP 2.1kPa = more than 20.5 g/g, or AAP 4.81 kPa = more than 20.5 g/g, water-soluble content = less than 10%, residual monomers = less than 500 ppm.

CRC = more than 30.0 g/g and 50 g/g or less, AAP 4.81 kPa = more than 20.5 g/g and 4050 g/g or less, water-soluble content = 4.0% or more and less than 8.3%, residual monomers = 50 ppm or more and less than 500 ppm.

### Applications of Water-Absorbent Resin of Present Invention

The application of the water-absorbent resin is not particularly limited, but is for hygienic materials. That is, in the present invention, a hygienic material containing a water-absorbent resin is provided. The hygienic material is preferably used for absorbers of absorbent articles such as disposable diapers (for infants and for adults), sanitary napkins, and incontinence pads. In particular, the water-absorbent resin can be used as an absorber of a high-density disposable diaper. Examples of other absorbent articles include drip absorbing materials, freshness retaining materials, portable toilets for disasters, absorbent sheet for pets, and cat sand. The water-absorbent resin is also used for soil water retaining agents, seedling-raising sheets, seed-coating materials, condensation prevention sheets, disposable heat packs, cooling bandanas, ice packs, liquid medical waste solidifying agents, residual soil solidification materials, dehydration agents for unpacked materials containing water, liquid waste gelling agents for water loss prevention, water-absorbing sandbags, poultice materials, thickeners for cosmetics, water-blocking materials for electrical and electronic materials and communication cables, gasket packings, sustained-release agents for fertilizers, various sustained-release agents (space disinfectants, air fresheners, etc.), dressing materials for wound protection, construction materials for condensation prevention, agents for removing moisture from oil, paints, adhesives, anti-blocking agents, light-diffusing agents, matting agents, and additives for decorative panels, additives for artificial marble, additives for resins such as additives for toner, and the like.

### Examples

### Production Example 1

As bioethanol, "Specific Ethanol Traceable 95 1st Grade" available from Japan Alcohol Trading CO., LTD. (Quality standard: 95.2 to 95.4 vol.% of ethanol content(equivalent to about from 92.6 to 92.8 wt.% (converted from a specific weight at 20°C)), with the remaining major portion being water, evaporation residue being 0.5 mg or less/100 ml, and including 30 mg or less/l of 2-propanol, 1 mg or less/l of each of methanol, 1-propanol, 1-butanol, 2-butanol, 2-methyl-1-propanol, 2-methylbutanol, and acetone, 2 mg or less/l of other organic impurities, less than 1 mg/l of acetaldehyde, and 0.01 mg/l or less of 1,4-dioxane) was used.

As the step (i) in the present invention, acetone was synthesized by reacting a gas of bioethanol/water vapor/nitrogen at 2/8/1 (molar ratio) at 400°C in the presence of a composite metal oxide catalyst having a composition of Fe/Zn/Zr at a molar ratio of 1/0.5/0.5, and purified to obtain acetone having a purity of 95 mass% or more. A total of 4000 ppm of ethanol and acetaldehyde were contained as impurities. The remaining major portion was water.

Next, as the step (ii), a gas of hydrogen/the acetone having a purity of 95 mass% or more at 2.7/1 (molar ratio) was reacted at 0.5 MPa and 100°C in the presence of a catalysts produced by supporting 5 wt.% of nickel and 5 wt.% of ruthenium on spherical silica (particle size: 1.7 to 4 mm) to synthesize isopropanol, thereby producing isopropanol having a purity of 98 mass%. The content of ethanol in isopropanol was 2200 ppm. The remaining major portion was water and acetone.

Further, as the step (iii), a gas of the isopropanol having a purity of 98 mass%/oxygen/nitrogen at 6.8/12.5/80.7 vol% was reacted at 325°C in the presence of a catalyst produced by supporting 10 wt.% of tungsten oxide on spherical γ-alumina with a particle size of 2 to 4 mm to obtain propylene.

Subsequently, as the step (iv), the propylene was oxidized at 325°C in the presence of a bismuth molybdenum-based catalyst (catalyst for acrolein) with a particle size of 5 to 7 mm to obtain acrolein, and subsequently, the acrolein was reacted at 275°C in the presence of a molybdenum vanadium-based catalyst with a particle size of 5 to 7 mm to obtain acrylic acid. This was purified to obtain acrylic acid (bioacrylic acid) having a purity of 99 mass% or more. The contents of acetic acid and propionic acid in the bioacrylic acid were 140 ppm and 200 ppm, respectively. The moisture content was 1500 ppm. The amount of an acrylic acid dimer was 80 ppm.

### [Comparative Production Example 1] Production of Acrylic Acid Derived from Fossil Raw Material

Instead of the method for producing biopropylene from bioethanol in the steps (i) to (iii) of Production Example 1, acrylic acid was produced by performing catalytic gas phase oxidation in the step (iv) using propylene derived from a fossil raw material, as a known method.

In absorption of the obtained reaction gas into water in an absorption column, hydroquinone was added and absorbed into the absorption liquid in an amount of 300 ppm with respect to an aqueous acrylic acid solution produced, thereby obtaining an aqueous acrylic acid solution. Next, components having a low boiling point, such as acetic acid and propionic acid, were removed from the aqueous acrylic acid solution by distillation using toluene as an azeotropic solvent, and components having a high boiling point, such as maleic acid and a dimer of acrylic acid were removed to obtain crude acrylic acid. The crude acrylic acid was purified with a simple distillation apparatus to obtain acrylic acid (2). The contents of acetic acid and propionic acid in the acrylic acid were 170 ppm and 210 ppm, respectively.

### [Comparative Production Example 2] Production of Acrylic Acid from Glycerin

Glycerin derived from a natural product was dehydrated in the presence of a strong acid solid catalyst to obtain acrolein containing by-products such as propanal. Next, acrolein containing propanal and the like was subjected to gas phase oxidation to obtain gaseous acrylic acid, which was collected with water to obtain an aqueous acrylic acid solution. The aqueous acrylic acid solution was distilled to obtain acrylic acid containing 3 wt.% of propionic acid. For further purifying the acrylic acid, simple distillation and crystallization were performed to obtain acrylic acid (3) derived from a non-fossil raw material and containing 2000 ppm of propionic acid and 300 ppm of acetic acid.

### [Example 1] Production of Water-Absorbent Resin from Acrylic Acid Derived from Bioethanol The step (v) of producing polyacrylic acid salt by polymerizing an aqueous monomer solution containing acrylic acid and a salt thereof

A polypropylene container having a volume of 2 L was charged with 439.4 parts by weight of bioacrylic acid (containing 80 ppm of an acrylic acid dimer, with 70 ppm of p-methoxyphenol incorporated separately) produced by carrying out the steps (i) to (iv) in Production Example 1, 181.1 parts by weight of 48.5 wt.% sodium hydroxide aqueous solution, 1.9 parts by weight of polyethylene glycol diacrylate (average number of polyethylene glycol units (average number): 9), 1.35 parts by weight of a 2.0 wt.% trisodium diethylenetriaminepentaacetate aqueous solution, and 351.7 parts by weight of deionized water, which were mixed to prepare an aqueous solution. The deionized water was heated to 40°C in advance.

Subsequently, 196.1 parts by weight of a 48.5 wt.% sodium hydroxide aqueous solution was added to the aqueous solution over about 30 seconds in a state of being open to the air while the aqueous solution was stirred, and mixing was performed to prepare an aqueous monomer solution. The temperature of the aqueous monomer solution was increased to about 80°C by the heat of neutralization and the heat of dissolution which were generated in the process of the mixing.

Thereafter, at an aqueous monomer solution temperature of 78°C, 28.45 parts by weight of a 3 wt.% aqueous sodium persulfate solution was added as a polymerization initiator, and the mixture was stirred for about 5 seconds to obtain a reaction solution.

Next, the reaction solution was poured into a stainless steel tray-shaped container in a state of being open to the air. The tray-shaped container had a bottom surface size of 200 mm × 260 mm, a top surface size of 460 mm × 560 mm, and a height of 140 mm. In the tray-shaped container, the cross-section of the central portion had a trapezoidal shape, and a silicone sheet attached to the inner surface. Before the reaction solution was poured, the tray-shaped container was placed on a hot plate heated to 50°C and preheated.

A polymerization reaction started within 1 minute after the reaction solution was poured into the tray-shaped container. In the polymerization reaction, the reaction solution upward expanded and foamed in all directions while generating water vapor as the polymerization reaction proceeded, followed by shrinkage to a size which is slightly larger than that of the bottom surface of the tray-shaped container. The polymerization reaction (expansion, shrinkage) was completed within about 1 minute. The maximum polymerization temperature as polymerization heat was 112°C, and a part of acrylic acid, acetic acid, and propionic acid remaining in the reaction system during the polymerization was vaporized and removed together with the generated water vapor. A crosslinked hydrogel polymer (hereinafter, referred to as "hydrogel") was produced by the above polymerization reaction.

Next, the hydrogel was cut into an appropriate size, and then supplied to a screw extruder, and the gel was ground (gel grinding step) to obtain a particulate hydrogel having a particle size of 0.1 to 2 mm. The moisture content of the particulate hydrogel was 52 wt.%.

### Step (vi) of Drying Hydrogel Polymer after Polymerization, and Step (vii) of Further Surface-Crosslinking

Next, as the step (vi), the particulate hydrogel was spread on a metallic wire mesh with a mesh size of 300 µm (50 mesh), and placed in a hot air dryer. Thereafter, the particulate hydrogel was dried by passage of hot air at 190°C for 30 minutes to obtain a dried polymer. The moisture content of the dried polymer was 2 wt.%. The particulate hydrogel discharged from the screw extruder was subjected to the step (vi) of drying within 1 hour. Subsequently, the dried polymer was put into a roll mill and ground, and classification was performed with two types of JIS standard sieves with mesh sizes of 850 µm and 150 µm to obtain water-absorbent resin powder (1) having an irregularly crushed shape. The weight average particle size (D50) of the water-absorbent resin powder (1) was 390 µm.

Next, as the step (vii), 3.5 parts by weight of a surface-crosslinking agent solution (1) including 0.4 parts by weight of ethylene carbonate, 0.6 parts by weight of propylene glycol, and 2.5 parts by weight of deionized water was added to 100 parts by weight of the water-absorbent resin powder (1), and mixing was performed until the mixture became homogeneous, thereby producing a humidified mixture (1). Subsequently, the humidified mixture (1) was heated at 200°C for 40 minutes, then cooled to 60°C, and allowed to pass through a sieve with a mesh size of 850 µm. Particles that had not passed through the sieve with a mesh size of 850 µm were crushed by lightly pressing the particles with a spatula on the mesh, and allowed to pass through the sieve, and particles that were not crushed enough were removed. In this way, a surface-crosslinked water-absorbent resin (1) having an irregularly crushed shape. Table 1 shows physical properties of the water-absorbent resin powder (1) and the water-absorbent resin (1).

### Comparative Example 1 Production of Water-Absorbent Resin with Acrylic Acid Derived from Fossil Raw Material

Except that in the step (v) of Example 1, acrylic acid produced from propylene derived from a fossil raw material in Comparative Production Example 1 was used, the same operation as in Example 1 was carried out to obtain comparative water-absorbent resin powder (1) having an irregularly crushed shape and a comparative surface-crosslinked water-absorbent resin (1).

### Comparative Example 2 Production of Water-Absorbent Resin with Acrylic Acid Derived from Glycerin

Except that in the step (v) of Example 1, acrylic acid produced in Comparative Production Example 2 was used, the same operation as in Example 1 was carried out to obtain comparative water-absorbent resin powder (2) having an irregularly crushed shape and a surface-comparative crosslinked water-absorbent resin (2).

### Comparative Example 3 Production of Water-Absorbent Resin Using Natural Polymer

Except that in Comparative Example 1 (production of acrylic acid derived from a fossil raw material), solubilized starch (25 wt.% per solid content of the monomer) was mixed with the hydrogel after polymerization in the step (vi) of Comparative Example 1 for producing a water-absorbent resin, a part of which is derived from a biological raw material, the same operation as in Comparative Example 1 was carried out to obtain a comparative water-absorbent resin (3) (starch content of 20 wt.%). The water-absorbent resins of Example 1 and Comparative Examples 1 and 2 were white. On the other hand, in the comparative water-absorbent resin (3) containing 20 wt.% of starch as shown in Comparative Example 1, the low heat resistance of starch led to a decrease in fluid retention capacity and coloration to ocher through drying (for 30 minutes with hot air at 190°C), and further, browning of the water-absorbent resin through surface-crosslinking (heat treatment at 200°C for 40 minutes) after drying.

### Comparative Example 4

For suppressing coloration and a decrease in fluid retention capacity through drying and surface-crosslinking in Comparative Example 3 (20% of starch caused coloration), the drying conditions (30 minutes with hot air at 190°C) were changed to four hours with hot air at 100°C in the step (vi) of Comparative Example 3. The coloration during drying was suppressed, the drying time increased, and a significant decrease in productivity was confirmed.

Further, for suppressing coloration through surface-crosslinking (heat treatment at 200°C for 40 minutes) after drying, the surface-crosslinking temperature was changed to 100°C, but even when the heating time was 1 hour, surface-crosslinking did not proceed, and AAP was low. In "Comparative Example 4" shown in Table 1, results when surface-crosslinking was performed under conditions of 100°C and 1 hour are shown.

### Comparative Example 5

In Comparative Example 3, for suppressing coloration through surface-crosslinking (heat treatment at 200°C for 40 minutes) after drying, 0.1 parts of ethylene glycol diglycidyl ether having high reactivity at low temperature was added as a surface-crosslinking agent to 0.4 parts by weight of ethylene carbonate and 0.6 parts by weight of propylene glycol, and surface-crosslinking was performed at 100°C for 1 hour.

**[Table 1]**

| | Proportion of biological raw material | CRC (g/g) | AAP0.7 (g/g) | Soluble content (wt.%) | Residual monomer (ppm) | Coloration | Total amount (ppm) of residual acetic acid and propionic acid |
|---|---|---|---|---|---|---|---|
| Example 1 | 100 | 30.2 | 25.0 | 8.2 | 440 | White | 240 |
| Comparative Example 1 | 0 | 30.0 | 24.9 | 8.3 | 430 | White | 260 |
| Comparative Example 2 | 100 | 31.0 | 23.9 | 10.0 | 500 | White | 1400 (acid odor) |
| Comparative Example 3 | 20 | 20.0 | 20.5 | 4.0 | 1200 | Brown | 200 |
| Comparative Example 4 | 20 | 27.5 | 11.5 | 8.3 | 900 | Light brown | 310 |
| Comparative Example 5 | 20 | 21.0 | 18.5 | 5.0 | 1180 | Yellow | 200 |

### [Example 2] Recycling of Water-Absorbent Resin

The fine powder passing through a 150 µm sieve when the dried polymer obtained by drying in the step (vi) in Example 1 was classified using two types of JIS standard sieves with mesh sizes of 850 µm and 150 µm in Example 1 was mixed in an amount of 1 wt.% (per the amount of the monomer used in the step (v)) in the gel grinding step of Example 1 to recycle the water-absorbent resin fine powder derived from a biological raw material. Almost the same water-absorbent resin as that of Example 1 was produced.

### [Example 3] Recycling of Acrylic Acid

In polymerization step in the step (v) in Example 1 (the maximum polymerization temperature as polymerization heat was 112°C, and a part of acrylic acid, acetic acid and propionic acid remaining in the reaction system during the polymerization was vaporized together with the generated water vapor), the vaporized acrylic acid was cooled and collected. The collected acrylic acid was purified, and used in an amount of 1 wt.% (per the amount of the monomer used in the step (v)) in polymerization in Example 1 to recycle the bioacrylic acid. Almost the same water-absorbent resin as that of Example 1 was produced.

### Outline

Comparison between Example 1 (the water-absorbent resin (1) of the present invention obtained with the acrylic acid produced from bioethanol and Comparative Example 1 (the known comparative water-absorbent resin (1) with acrylic acid produced from a fossil raw material) shows that the production method of the present invention ensures equivalent performance and an equivalent or reduced amount of impurities (acetic acid and propionic acid which cause odors) as compared to a known water-absorbent resin derived from a fossil raw material.

Comparison between Example 1 (the water-absorbent resin (1) of the present invention with the acrylic acid produced from bioethanol) and Comparative Example 2 (the comparative water-absorbent resin (2) with acrylic acid produced from bio glycerin) shows that the production method of the present invention ensures excellent water absorption performance (relationship between the fluid retention capacity and the soluble content) and a reduced amount of impurities (acetic acid and propionic acid). In the water-absorbent resin of Comparative Example 2 (residual acetic acid/propionic acid at 1400 ppm), it was difficult to sufficiently remove impurities such as propionic acid (boiling point of 141°C) in acrylic acid (boiling point of 141°C) by purification even when the acrylic acid was highly purified at the sacrifice of the yield and cost in Comparative Production Example 2, and thus generation of an acid odor from the obtained water-absorbent resin during storage was confirmed in addition to problems in purification cost and the yield of acrylic acid.

Comparison between Example 1 (340 ppm in total of residual acetic acid/propionic acid in acrylic acid) and Comparative Example 2 (2300 ppm in total of residual acetic acid and propionic acid in acrylic acid) shows that Example 1 is superior in yield of the water-absorbent resin (ratio of acrylic acid used and water-absorbent resin obtained) to Comparative Example 2 in which the amount of impurities in the acrylic acid is large, so that a larger amount of acetic acid and propionic acid is vaporized.

Comparison of Example 1 (the water-absorbent resin (1) of the present invention with acrylic acid produced from bioethanol), Comparative Example 1 (the known comparative water-absorbent resin (1) with acrylic acid produced from a fossil raw material), and Comparative Examples 3 to 5 (the comparative water-absorbent resin (3) (the proportion of the biological raw material: 20 wt.%) as a starch graft produced from acrylic acid produced from a fossil raw material and starch (25 wt.% per the solid content of the monomer) shows that the use of starch with a polyacrylic acid salt reduces the centrifuge retention capacity (CRC) of the water-absorbent resin, increases the amount of residual monomers, and changes the color of the water-absorbent resin from yellow to brown. Since the water-absorbent resin is mainly used for hygienic materials such as diapers, a decrease in centrifuge retention capacity (CRC), or coloration of the water-absorbent resin and an increase in the amount of residual monomer as in Comparative Examples 3 to 5 are not preferred.

By recycling bioacrylic acid and a water-absorbent resin derived from a biological raw material as in Examples 2 and 3, a more carbon-neutral water-absorbent resin having higher performance can be provided.

From the water-absorbent resin of Example 1 in which acrylic acid produced from hydrous ethanol containing predetermined minor components in Production Example 1 is used, it can be seen that the remaining of water or isopropanol in ethanol as bioethanol used does not have an adverse effect on acrylic acid used in the water-absorbent resin. As ethanol used in the present invention, hydrous ethanol which is relatively inexpensive and is free of a hydrophobic solvent, in particular, hydrous ethanol (and further, crude ethanol) containing a fermentation byproduct (isopropanol or acetone) or water can be appropriately used instead of anhydrous ethanol which requires a hydrophobic solvent for a high level of purification, and it can be confirmed that results similar to those in Example 1 are obtained.

That is, it can be seen that the water-absorbent resin from the production method of Examples of the present invention, in which substantially 100 wt.% of the backbone is derived from a biological raw material, is superior in water absorption performance (CRC/AAP) and residual monomers to comparative water-absorbent resins (3) to (5) derived from 20% starch, and is white despite being produced at a high temperature.

### Industrial Applicability

By producing a water-absorbent resin using bioethanol as a raw material, a carbon-neutral water-absorbent resin having high performance can be provided. Since bioethanol is a biological raw material that is inexpensive and is produced in a large amount, water-absorbent resins produced from bioethanol by the production method of the present invention can be widely used in the field of application of water-absorbent resins in place of known water-absorbent resins derived from fossil raw materials which are consumed in a large amount.

The present application is based on JP 2023-198629 A filed on November 22, 2023 and JP 2023-223208 A filed on December 28, 2023, the disclosure of which is incorporated herein by reference in its entirety.

## Claims

1. A method for producing a water-absorbent resin derived from a biological raw material, the method comprising the following steps (i) to (vii) of:
(i) producing acetone from bioethanol;
(ii) producing isopropanol from the acetone;
(iii) producing propylene from the isopropanol;
(iv) producing acrylic acid from the propylene;
(v) producing polyacrylic acid and/or a salt thereof by polymerizing an aqueous monomer solution containing the acrylic acid;
(vi) drying the polyacrylic acid and/or a salt thereof; and
(vii) performing surface-crosslinking on the polyacrylic acid and/or a salt thereof.

2. The production method according to claim 1, wherein the acetone has a total content of ethanol and acetaldehyde of 20000 ppm or less.

3. The production method according to claim 1 or 2, wherein the isopropanol has an ethanol content of 20000 ppm or less.

4. The production method according to any one of claims 1 to 3, wherein the bioethanol is hydrous ethanol having a moisture content of 3 wt.% or more.

5. The production method according to any one of claims 1 to 4, wherein a content of each of a lower alcohol having 1 or 3 to 5 carbon atoms, acetaldehyde, and acetone in the bioethanol is 1 wt.% or less.

6. The production method according to any one of claims 1 to 5, wherein the bioethanol is ethanol containing isopropanol and/or acetone.

7. The production method according to any one of claims 1 to 6, wherein the bioethanol is produced by fermentation of one or more genetically modified or non-genetically modified plant raw materials selected from sugar cane, corn, and sugar beet.

8. The production method according to any one of claims 1 to 7, wherein a content of propionic acid in the acrylic acid is 500 ppm or less.

9. The production method according to any one of claims 1 to 8, wherein the acrylic acid has a content of acetic acid of 1500 ppm or less.

10. The production method according to any one of claims 1 to 9, wherein at least a part of impurities in the acrylic acid is removed in the step (v) and/or the step (vi).

11. The production method according to any one of claims 1 to 10, wherein acrylic acid vaporized in the step (v) and/or the step (vi) is recycled to the step (v).

12. The production method according to any one of claims 1 to 11, wherein in the step (v) and subsequent steps, a part of the water-absorbent resin is separated and recycled to the step (v) or the step (vi).

13. The production method according to any one of claims 1 to 12, wherein in the step (v), in addition to the acrylic acid produced in the step (iv), additional acrylic acid is used in combination in a monomer, and a content of the acrylic acid produced in the step (iv) is 1 mol% or more with respect to a total amount of acrylic acid.

14. The production method according to any one of claims 1 to 13, wherein the water-absorbent resin has a total content of acetic acid and propionic acid of 1000 ppm or less.

15. The production method according to any one of claims 1 to 14, wherein the water-absorbent resin has a total content of acetic acid, propionic acid, and acrylic acid of 1500 ppm or less.

16. The production method according to any one of claims 1 to 15, wherein the water-absorbent resin has a ratio of ¹⁴C/¹²C of 1.0 × 10⁻¹⁴ or more as measured by radiocarbon dating.

17. The production method according to any one of claims 1 to 16, wherein in the step (v) and subsequent steps, a chelating agent is added to the aqueous monomer solution, and/or the polyacrylic acid and/or a salt thereof.

18. The production method according to claim 6, wherein the bioethanol has a total content of acetone and isopropanol of 1 ppm or more.

19. A water-absorbent resin produced by the production method described in any one of claims 1 to 18.

20. A hygienic material comprising the water-absorbent resin described in claim 19.
